# EUROPEAN PATENT APPLICATION

(11) **EP 3 093 284 A1**
(43) Date of publication of application: **16.11.2016**
(21) Application number: 15734925.9
(22) Date of filing: 07.01.2015
(51) Int. Cl.: C07D 231/14, A01N 43/56, A01N 47/18, A01P 3/00, C07D 401/04

(54) **ETHEREAL OXYGEN-CONTAINING PERFLUOROALKYL GROUP-SUBSTITUTED PYRAZOLE COMPOUND AND PRODUCTION METHOD THEREFOR**

(30) Priority: 10.01.2014 JP 2014003589
(71) Applicant: Asahi Glass Company, Limited, Tokyo 100-8405 (JP)
(72) Inventor: MORIZAWA, Yoshitomi, Tokyo 100-8405 (JP); TAKAHIRA, Yusuke, Tokyo 100-8405 (JP)
(74) Representative: Müller-Boré & Partner Patentanwälte PartG mbB
(86) International application number: PCT/JP2015/050286
(87) International publication number: WO 2015/105129

(57) **Abstract**

To provide a pyrazole derivative having a superior fluorinated substituent, which is useful for pharmaceutical products or agricultural chemicals having pharmacological activities, and a method for its production. A compound represented by the hollowing formula (A), wherein R^{f} is a C₂₋₁₉ perfluoroalkyl group having ethereal oxygen atom(s) inserted between carbon-carbon atoms of the group, and the total number of carbon atoms and oxygen atoms in R^{f} being from 3 to 20, one of Q¹ and Q² is a hydrogen atom, and the other is a phenyl group, or a phenyl group in which at least one hydrogen atom in the phenyl group is each independently substituted by a halogen atom, and one of A¹ and A² is a nitrogen atom, and the other is a nitrogen atom to which R¹ is bonded.

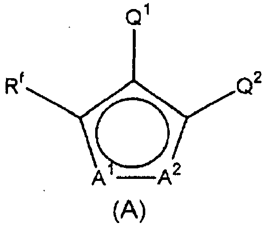

## Description

### TECHNICAL FIELD

The present invention relates to a pyrazole compound substituted by an ethereal oxygen atom-containing perfluoroalkyl group, and a method for producing such a compound.

### BACKGROUND ART

Compounds having a heterocyclic ring (hereinafter referred to as "heterocyclic compounds") are often found in natural products or biogenic substances and have been often used as pharmacologically active substances in pharmaceuticals, pesticides, etc. Further, they are widely used as functional materials such as liquid crystal materials, organic semiconductor materials, etc. Especially, it is possible to construct a variety of structures by selecting the combination of the types of heterocyclic rings, the number and positions of substituents, and the presence or absence of aromaticity. Among them, heterocyclic compounds having a fluorinated substituent, utilizing the unique properties specific to fluorine atoms, are practically used in many cases as medicines and agricultural chemicals having excellent pharmacological activities.

In pharmaceuticals and agricultural chemicals, most of fluorinated substituents of heterocyclic compounds used to be fluorine atoms or perfluoroalkyl groups. In recent years, 4-heptafluoro-isopropyl-2-(trifluoromethylthio) aniline, flubendiamide, etc. have been reported as agricultural chemicals having a trifluoromethyl group or a heptafluoro-2-propyl group as a perfluoroalkyl group (Patent Document 1 and Non-patent Document 1).

Among heterocyclic compounds, pyrazole derivatives having a pyrazole ring being a 5 membered ring wherein two nitrogen atoms are adjacent to each other, are useful as pharmaceuticals, agricultural chemicals, or intermediates for their production, and a number of compounds have been known. As a pyrazole derivative having a fluorinated substituent, a 5-trifluoromethylpyrazole-4-carboxylic acid derivative or a 5-pentafluoroethylpyrazole-4-carboxylic acid derivative to be synthesized by a reaction represented by the following formulae, is known (Patent Document 2).

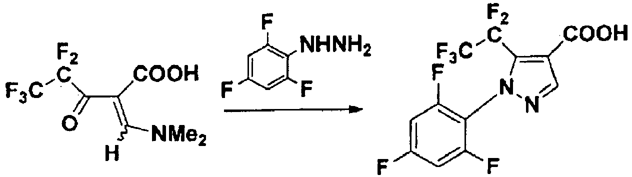

As a compound having a nitrogen-containing 5-membered heterocyclic ring other than a pyrazole derivative and having a fluorinated substituent group containing an ethereal oxygen atom, a compound represented by the following formula is known as an imidazole derivative (Patent Document 3).

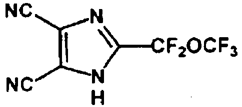

As a pyrimidine derivative, 2-perfluoro(2-methoxy(ethoxy)methyl)-4,6-bistrifluoromethyl-5-fluoropyrimidine represented by the following formula is known (Non-patent Document 2).

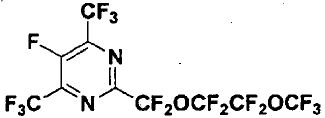

Among pyrazole derivatives, as a compound substituted by an ethereal oxygen atom-containing alkyl group, a method for producing the following pyrazole derivative substituted by four substituents including a perfluoro(methoxymethyl) group is known (Non-patent Document 3).

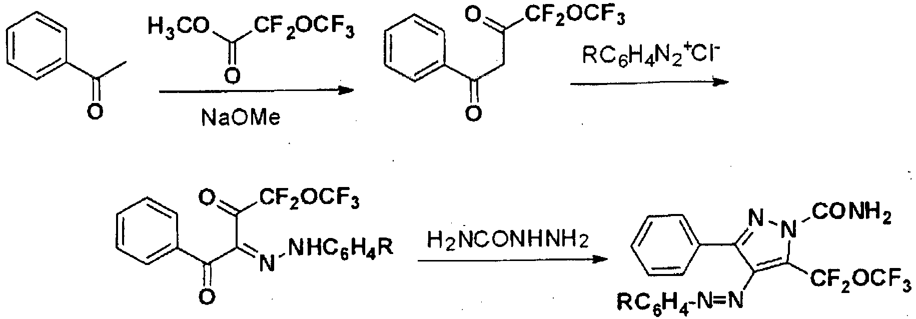

Further, the following compound has been reported as a pyrazole substituted by a perfluoro(2-methoxyethoxymethyl) group (Patent Document 4).

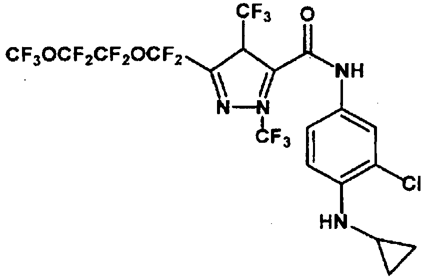

### PRIOR ART DOCUMENTS

### PATENT DOCUMENTS

Patent Document 1: WO2006/137395
Patent Document 2: JP-A-2010-202648
Patent Document 3: WO2013/072591
Patent Document 4: WO2010/051926

### NON-PATENT DOCUMENTS

Non-patent Document 1: Fine Chemicals, Vol. 36, No. 8, pp. 58-65
Non-patent Document 2: Fluorine Notes (2009), p. 65
Non-patent Document 3: Ukrainskii Khimisheskii Zhurnal, (1981) 47, pp. 1,078-1,085

### DISCLOSURE OF INVENTION

### TECHNICAL PROBLEM

However, a compound with a pyrazole ring has not been known in which to two carbon atoms among the three carbon atoms of the pyrazole ring, a fluorinated alkyl group having an ethereal oxygen atom and a specific substituent (one of the following groups (1) to (7) of the present invention) are bonded, and to the remaining one carbon atom, a hydrogen atom is bonded.

It is an object of the present invention to provide a novel pyrazole derivative and a method for its production.

### SOLUTION TO PROBLEM

The present inventors have found, as a novel compound, a compound with a pyrazole ring in which to two carbon atoms among the three carbon atoms of the pyrazole ring, a fluorinated alkyl group having an ethereal oxygen atom and a specific substituent are bonded, and to the remaining one carbon atom, a hydrogen atom is bonded, and thus have accomplished the present invention.

That is, the gist of the present invention is as follows.
<1> A compound represented by the following formula (A): 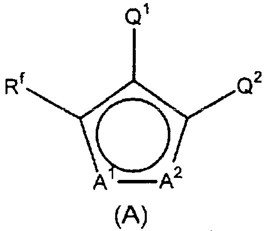 in the above formula, R^{f}, A¹, A², Q¹ and Q² have the following meanings, respectively:
   R^{f}: a C₂₋₁₉ perfluoroalkyl group in which ethereal oxygen atom(s) is inserted between carbon-carbon atoms of the group, and the sum of the number of carbon atoms and the number of oxygen atom(s) in said Rf is from 3 to 20,
   A¹ and A²: either one is a nitrogen atom, and the other is a nitrogen atom to which R¹ is bonded, wherein R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group is each independently substituted by a group selected from a hydroxy group, an amino group, a carboxy group and a halogen atom, (vi) a group selected from the above groups (ii) to (iv), in which at least one hydrogen atom bonded to a carbon atom of the group is each independently substituted by a C₁₋₆ halogenated alkyl group, (vii) a hydrogen atom, or (viii) a group represented by R¹²C(O)- wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group, or an aralkyl group) is inserted between carbon-carbon atoms in the alkyl group, (c) a C₁₋₆ alkoxy group, (d) a C₁₋₆ alkoxy group in which an oxygen atom is inserted between carbon-carbon atoms of the alkoxy group, (e) a phenyl, or (f) a group selected from the above groups (a) to (e), in which at least one hydrogen atom bonded to a carbon atom of the group is each independently substituted by a group selected from a hydroxy group, an amino group, a carboxy group and a halogen atom,
   Q¹ and Q²: either one is a hydrogen atom, and the other is one group selected from the following (1) to (7):
      (1) a phenyl group, or a phenyl group in which at least one hydrogen atom of the phenyl group is each independently substituted by a group selected from a hydroxy group, an amino group, a carboxy group, a halogen atom and a trifluoromethyl group,
      (2) a group represented by R¹⁷C(O)- (wherein R¹⁷ is a C₁₋₆ alkyl group, a phenyl group, or a C₁₋₆ alkyl group or a phenyl group, in which at least one hydrogen atom bonded to a carbon atom of the group, is each independently substituted by a group selected from an amino group, a hydroxy group, a halogen atom, a C₁₋₆ alkoxy group and a trifluoromethyl group),
      (3) a group represented by R⁷OC(O)- (wherein R⁷ is a C₁₋₆ alkyl group, an aralkyl group, a phenyl group, or a phenyl group in which at least one hydrogen atom of the phenyl group is each independently substituted by a group selected from an amino group, a substituted amino group, a C₁₋₆ alkoxy group and a halogen atom),
      (4) a carboxy group,
      (5) a group represented by R¹⁶OC(O)NH- (wherein R¹⁶ is a C₁₋₆ alkyl group, or a C₁₋₆ alkyl group in which at least one hydrogen atom bonded to a carbon atom of the group is substituted by a halogen atom),
      (6) an amino group,
      (7) a group represented by R²²C(O)NH- (wherein R²² is a C₁₋₆ alkyl group, a phenyl group, an aralkyl group, or a C₁₋₆ alkyl group, a phenyl group or an aralkyl group, in which at least one hydrogen atom bonded to a carbon atom of the group is each independently substituted by a group selected from an amino group, a hydroxy group, a halogen atom and a trifluoromethyl group).
   <2> The compound according to the above <1>, wherein in the formula (A), R^{f} is a group represented by (R^{f10})(R^{f11})(R^{f12})C-O-(R^{f13})(R^{f14})C-, wherein R^{f10} to R^{f14} are each independently a C₁₋₁₂ perfluoroalkyl group, a C₁₋₁₂ perfluoroalkyl group having an ethereal oxygen atom at least either between carbon-carbon atoms or at a bond terminal, or a fluorine atom, and the sum of the number of carbon atoms and the number of oxygen atoms, is from 3 to 20.
   <3> The compound according to the above <1> or <2>, wherein in the formula (A), A¹ is a nitrogen atom to which said R¹ is bonded, and A² is a nitrogen atom.
   <4> The compound according to the above <1> or <2>, wherein the compound represented by the formula (A) is a compound represented by the following formula (1 a) or a compound represented by the following formula (1 b): 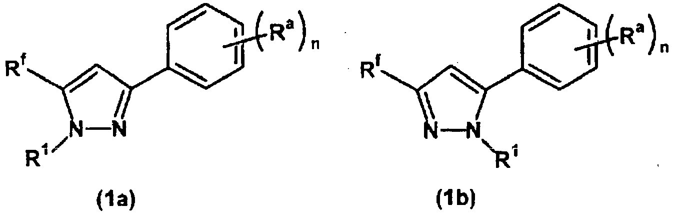 in the above formulae, the definitions of R^{f} and R¹ are the same as above, R^{a} is a hydroxy group, an amino group, a carboxy group, a halogen atom or a trifluoromethyl group, and n is an integer of from 0 to 5.
   <5> A method for producing at least one of a compound represented by the following formula (1 a) and a compound represented by the following formula (1 b), which is characterized by reacting a compound represented by the following formula (14) and a compound represented by the formula R¹NHNH₂: 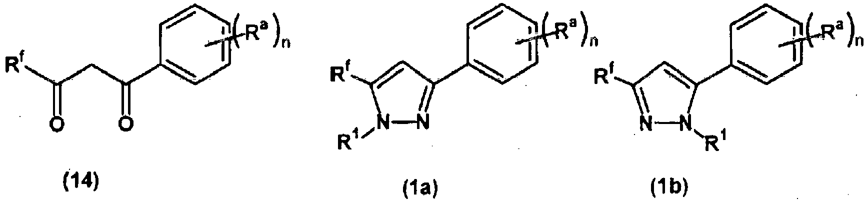 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, R^{a} is a hydroxy group, an amino group, a carboxy group, a halogen atom or a trifluoromethyl group, and n is an integer of from 0 to 5.
   <6> The compound according to the above <1> or <2>, wherein the compound represented by the formula (A) is a compound represented by the following formula (2a) or a compound represented by the following formula (2b): 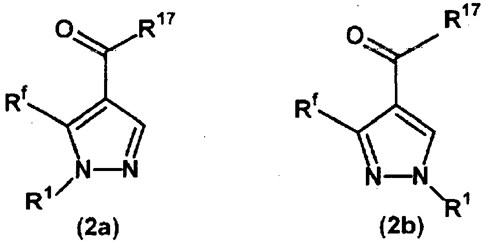 in the above formulae, the definitions of R^{f}, R¹ and R¹⁷ are the same as above.
   <7> A method for producing at least one of a compound represented by the following formula (2a) and a compound represented by the following formula (2b), which is characterized by reacting a compound represented by the following formula (16) and a compound represented by the formula R¹NHNH₂: 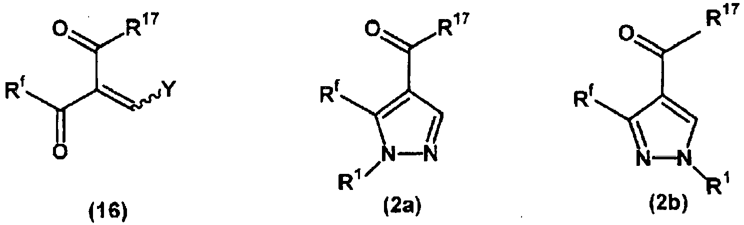 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, R¹⁷ is a C₁₋₆ alkyl group, a phenyl group, or a C₁₋₆ alkyl group or a phenyl group, in which at least one hydrogen atom bonded to a carbon atom of the group, is each independently substituted by a group selected from an amino group, a hydroxy group, a halogen atom, a C₁₋₆ alkoxy group and a trifluoromethyl group, Y is a group represented by -OR' or NR"₂, and the above R' and R" each independently represent a C₁₋₃ alkyl group.
   <8> The compound according to the above <1> or <2>, wherein the compound represented by the formula (A) is a compound represented by the following formula (3a) or a compound represented by the following formula (3b): 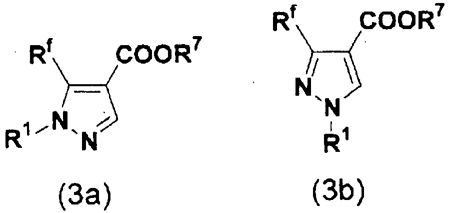 in the above formulae, the definitions of R^{f}, R¹ and R⁷ are the same as above.
   <9> A method for producing at least one of a compound represented by the following formula (3a) and a compound represented by the following formula (3b), which is characterized by reacting a compound represented by the following formula (19) and a compound represented by the formula R¹NHNH₂: 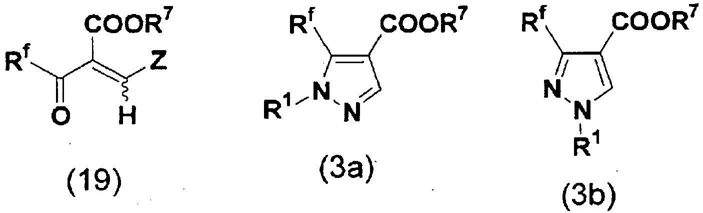 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R⁷ is a C₁₋₆ alkyl group, an aralkyl group, a phenyl group, or a phenyl group in which at least one hydrogen atom of the phenyl group is each independently substituted by a group selected from an amino group, a substituted amino group, a C₁₋₆ alkoxy group and a halogen atom, Z is a group represented by -OR' or NR"₂, and the above R' and R" each independently represent a C₁₋₃ alkyl group, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom.
   <10> The compound according to the above <1> or <2>, wherein the compound represented by the formula (A) is a compound represented by the following formula (4a) or a compound represented by the following formula (4b): 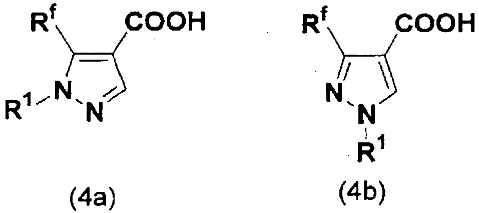 in the above formulae, the definitions of R^{f} and R¹ are the same as above.
   <11> A method for producing at least one of a compound represented by the following formula (4a) and a compound represented by the following formula (4b), which is characterized by hydrolyzing at least one of a compound represented by the following formula (3a) and a compound represented by the following formula (3b) under a basic condition: 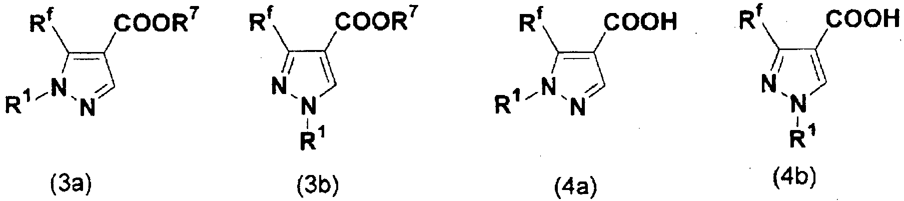 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R⁷ is a C₁₋₆ alkyl group, an aralkyl group, a phenyl group, or a phenyl group in which at least one hydrogen atom of the phenyl group is each independently substituted by a group selected from an amino group, a substituted amino group, a C₁₋₆ alkoxy group and a halogen atom.
   <12> The compound according to the above <1> or <2>, wherein the compound represented by the formula (A) is a compound represented by the following formula (5a) or a compound represented by the following formula (5b): 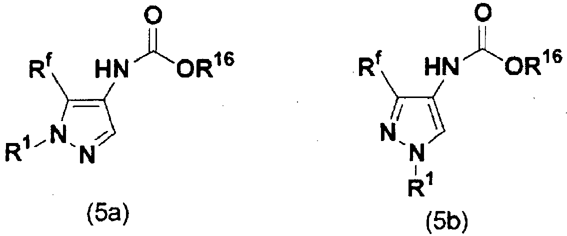 in the above formulae, the definitions of R^{f}, R¹ and R¹⁶ are the same as above.
   <13> A method for producing at least one of a compound represented by the following formula (5a) and a compound represented by the following formula (5b), which is characterized in that at least one of a compound represented by the following formula (4a) and a compound represented by the following formula (4b) is formed into a mixed acid anhydride, which is then converted by a metal azide compound to a keto azide, which is further reacted with a compound represented by R¹⁶-OH while being subjected to a rearrangement reaction: 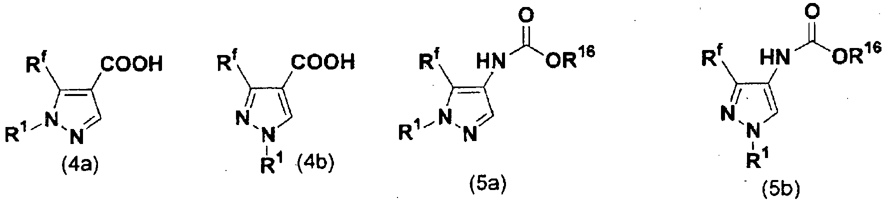 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R¹⁶ is a C₁₋₆ alkyl group, or a C₁₋₆ alkyl group in which at least one hydrogen atom bonded to a carbon atom of the alkyl group is substituted by a halogen atom.
   <14> The compound according to the above <1 > or <2>, wherein the compound represented by the formula (A) is a compound represented by the following formula (6a) or a compound represented by the following formula (6b): 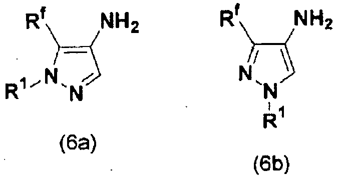 in the above formulae, the definitions of R^{f} and R¹ are the same as above.
   <15> A method for producing at least one of a compound represented by the following formula (6a) and a compound represented by the following formula (6b), which is characterized by reacting at least one of a compound represented by the following formula (5a) and a compound represented by the following formula (5b) with an acid: 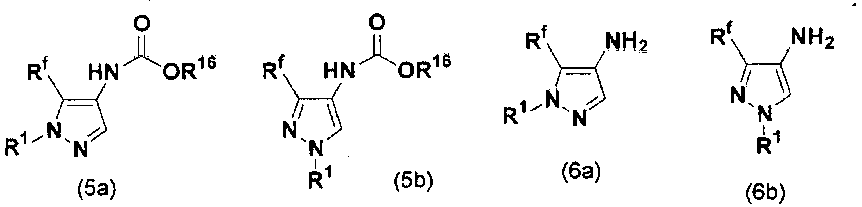 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R¹⁶ is a C₁₋₆ alkyl group, or a C₁₋₆ alkyl group in which at least one hydrogen atom bonded to a carbon atom of the alkyl group is substituted by a halogen atom.
   <16> The compound according to the above <1> or <2>, wherein the compound represented by the formula (A) is a compound represented by the following formula (7a) or a compound represented by the following formula (7b): 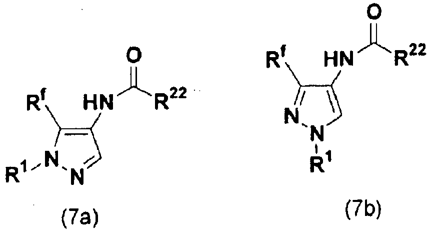 in the above formulae, the definitions of R^{f}, R¹ and R²² are the same as above.
   <17> A method for producing at least one of a compound represented by the following formula (7a) and a compound represented by the following formula (7b), which is characterized by reacting at least one of a compound represented by the following formula (6a) and a compound represented by the following formula (6b) with R²²COX (wherein X is a halogen atom, a group obtained by removing a hydrogen atom from N-hydroxysuccinimide, a C₁₋₆ alkoxy group, a perfluoro phenoxy group or a C₁₋₆ fluoroalkoxy group): 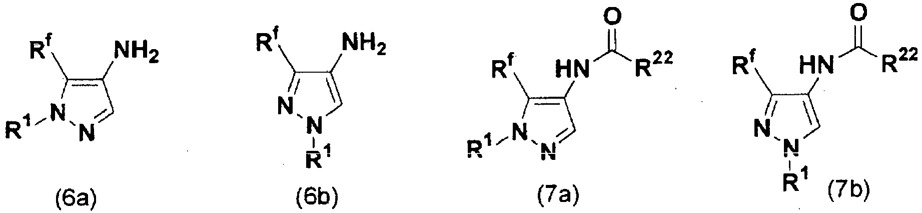 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R²² is a C₁₋₆ alkyl group, a phenyl group, an aralkyl group, or a C₁₋₆ alkyl group, a phenyl group or an aralkyl group, in which at least one hydrogen atom bonded to a carbon atom of the group, is each independently substituted by a group selected from an amino group, a hydroxy group, a halogen atom and a trifluoromethyl group.
   <18> An agricultural chemical containing a compound selected from the group consisting of compounds as defined in any one of the above <1> to <4>, <6>, <8>, <10>, <12>, <14> and <16> and the pharmacologically active salts of such compounds.

### ADVANTAGEOUS EFFECTS OF INVENTION

The compound of the present invention is a novel pyrazole derivative in which to two carbon atoms among the three carbon atoms of the pyrazole ring, a fluorinated alkyl group having an ethereal oxygen atom, and a specific substituent, are bonded, and to the remaining one carbon atom, a hydrogen atom is bonded. In a case where substituents in the compound of the present invention have an ester bond, an amide bond or a carboxy group, derivatization to various pyrazole compounds becomes possible by conversion reactions of such substituents, and therefore, the compound of the present invention is useful also as an intermediate for such pyrazole ring compounds. Further, the compound of the present invention is expected to have high pharmacological activities a drug substance for a novel medicine or agricultural chemical.

### DESCRIPTION OF EMBODIMENTS

Now, the present invention will be described in detail, but it should be understood that the present invention is by no means limited to the following embodiments, and can be practiced by optionally modifying them without departing from the scope of the present invention.

In this specification, a compound represented by the general formula (n) may be simply expressed as "compound (n)". A perfluoroalkyl group is meant for an alkyl group in which all of hydrogen atoms of the alkyl group are substituted by fluorine atoms.

The compound of the present invention is represented by the following formula (A).

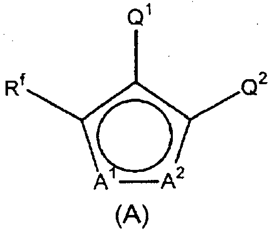

In the above formula (A), the definitions of R^{f}, A¹, A², Q¹ and Q² are respectively as described above.

In the formula (A), the sum of the number of carbon atoms and the number of oxygen atoms in R^{f} is preferably from 3 to 12, particularly preferably from 6 to 10. The number of carbon atoms in R^{f} is preferably from 2 to 10. The number of oxygen atoms is preferably 1 or 2. R^{f} may be either straight-chained or branched.

The compound (A) is a pyrazole derivative wherein a perfluoroalkyl group having an ethereal oxygen atom is bonded to a carbon atom on the pyrazole ring. The perfluoroalkyl group having an ethereal oxygen atom is a group capable of being bended and having a hydrophobic property, whereby when it is used as a pharmaceutical or agrochemical, it becomes possible to be bonded to an active site and to exhibit a physiological activity.

R^{f} is preferably a group represented by the following formula (23). In the formula (23), R^{f10} to R^{f14} are each independently a C₁₋₁₂ perfluoroalkyl group, a C₁₋₁₂ perfluoroalkyl group having an ethereal oxygen atom between carbon-carbon atoms, or a fluorine, and the sum of the number of carbon atoms and the number of oxygen atoms is from 3 to 20.

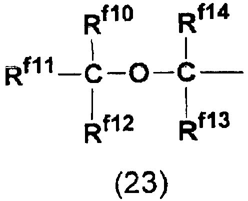

When each of R^{f10} to R^{f14} is a C₁₋₁₂ perfluoroalkyl group, it is preferably a group having from 1 to 8 carbon atoms, and a trifluoromethyl group, a pentafluoroethyl group, a heptafluoropropyl group, a heptafluoroisopropyl group, a nonafluorobutyl group, a nonafluoroisobutyl group, a perfluorohexyl group, a perfluorooctyl group, etc. may be mentioned as preferred examples.

When each of R^{f10} to R^{f14} is a C₁₋₁₂ perfluoroalkyl group having an ethereal oxygen atom, it is preferably a group having from 1 to 8 carbon atoms and from 1 to 3 ethereal oxygen atoms, and a perfluoro(methoxymethyl) group, a perfluoro(ethoxymethyl) group, a perfluoro(isopropoxymethyl) group, a perfluoro(1-methoxyethyl) group, a perfluoro(1-ethoxyethyl) group, a perfluoro((2-methoxy)ethoxymethyl) group, a perfluoro((2-ethoxy) ethoxy-methyl) group, a perfluoro(1-propoxyethyl) group, a perfluoro(1-(2-propoxy-2-methylethoxy) ethyl) group, etc. may be mentioned as preferred examples.

As R^{f10} to R^{f14}, R^{f10} is preferably a C₁₋₈ perfluoroalkyl group or a C₁₋₈ perfluoroalkyl group having an ethereal oxygen atom, wherein the number of ethereal oxygen atoms is from 1 to 3, and each of R^{f11} to R^{f14} is preferably a C₁₋₁₂ perfluoroalkyl group (preferably a trifluoromethyl group) or a fluorine atom.

As R^{f}, specifically groups of the following structures may be mentioned as particularly preferred groups.

CF₃CF₂CF₂OCF(CF₃)-

CF₃OCF₂CF₂OCF₂-

CF₃CF₂OCF₂CF₂OCF₂-

CF₃CF₂OCF₂-

The compound (A) is preferably a compound wherein A¹ is a nitrogen atom to which R¹ is bonded, and A² is a nitrogen atom.

R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group is each independently substituted by a group selected from a hydroxy group, an amino group, a carboxy group and a halogen atom, (vi) a group selected from the above groups (ii) to (iv), in which at least one hydrogen atom bonded to a carbon atom of the group is each independently substituted by a C₁₋₆ halogenated alkyl group, (vii) a hydrogen atom, or (viii) a group represented by R¹²C(O)-.

Examples of R¹ which is group (i): a C₁₋₆ alkyl group, include a methyl group, an ethyl group, a propyl group, an isopropyl group, a butyl group, a hexyl group, etc.

Examples of R¹ which is group (iii): a monovalent 5-membered ring group containing a hetero atom, or group (iv): a monovalent 6-membered ring containing a hetero atom, include a 2-thiophenyl group, a 3-thiophenyl group, a 2-pyridyl group, a 3-pyridyl group, a 4-pyridyl group, etc.

In a case where R¹ is group (v) or group (vi), the halogen atom as the substituent may be a fluorine atom, a chlorine atom or an iodine atom, preferably a fluorine atom. The C₁₋₆ halogenated alkyl group as the substituent is preferably a C₁₋₆ perfluoroalkyl group.

An example of R¹ which is group (v) or group (vi), is preferably a phenyl group substituted by at least one fluorine atom, a phenyl group substituted by at least one C₁₋₆ perfluoroalkyl group, or a phenyl group substituted by at least one fluorine atom and at least one C₁₋₆ perfluoroalkyl group. Specifically, a 2-fluorophenyl group, a 4-fluorophenyl group, a 2-trifluoromethylphenyl group, a 4-trifluoromethylphenyl group, a 2-fluoro-4-trifluoromethylphenyl group, a 2,6-difluoro-4-trifluoromethylphenyl group, a 2,4-difluorophenyl group, a 2,4,6-trifluoro-phenyl group, a 2,6-dichloro-4-trifluoromethylphenyl group, a 3-trifluoromethyl-2-pyridyl group, a 4-trifluoromethyl-2-pyridyl, etc. may be mentioned as preferred examples.

In a case where R¹ is group (viii): a group represented by R¹²C(O)-, (a) a C₁₋₆ alkyl group for R¹² may be the same group as the C₁₋₆ alkyl group for R¹. Examples of R¹² which is (b) a C₁₋₆ alkyl group wherein an oxygen atom, a sulfur atom or NR- (wherein R is a hydrogen atom, a C₁₋₃ alkyl group, a phenyl group or an aralkyl a group) is inserted between carbon-carbon groups, include a methoxymethyl group, an ethoxymethyl group, a methoxyethoxymethyl group, a 2,2,2-trifluoroethoxyethyl group, a methylthio group, an ethylthio group, a propylthio group, a phenylamino group, a benzylamino group, a 2-fluorobenzyl group, a (2-fluoro-4-trifluoromethylphenyl)methyl group, etc.

When R¹² is (c) a C₁₋₆ alkoxy group, the alkyl group moiety of the alkoxy group is not particularly limited, and may be either straight chained or branched. Examples of the C₁₋₆ alkoxy group include a methoxy group, an ethoxy group, a n-propyloxy group, an i-propyloxy group, a n-butyloxy group, a s-butyloxy group, a t-butyloxy group, etc.,

When R¹² is (d) a C₁₋₆ alkoxy group wherein an oxygen atom is inserted between carbon-carbon atoms of the group, it may be an alkoxyalkoxy group. Specifically, a methoxymethoxy group, a methoxyethoxy group, an ethoxyethoxy group, a methoxy(ethoxy)ethoxy group, etc. may be mentioned.

When R¹ is (f) a group selected from the groups (a) to (e), wherein at least one hydrogen atom bonded to a carbon atom of the group is substituted, it is preferably a group substituted by a halogen atom.

R¹ is preferably a C₁₋₆ alkyl group, a phenyl group, a pyridinyl group, or a phenyl group wherein at least one carbon atom is substituted by a halogen atom, and a fluorophenyl group, a fluoro(trifluoromethyl) phenyl group, a dichloro(trifluoromethyl)phenyl group, etc. are particularly preferred.

Of Q¹ and Q² in the formula (A), either one is a hydrogen atom, and the other is a group selected from the following (1) to (7). Q¹ and Q² will be described based on the synthesis scheme of the compound (A).

The compound (A) of the invention can be obtained by the following Route A or Route B using a compound of the formula R^{f}C(O)X as a raw material. However, the production routes of the compound (A) are not limited to the following routes.

Route A: A production route to obtain a compound (A) wherein Q¹ or Q² is a group (1) or a group (2) by using, as a raw material, an intermediate compound obtained by reacting a carbanion to a compound (13) represented by the formula R^{f}C(O)X.

Route B: A production route to sequentially obtain compounds wherein Q¹ or Q² is one of groups (3) to (7), by using, as a raw material, an intermediate (compound (19)) obtained by reacting a compound represented by the formula (18) to a compound (13) represented by the formula R^{f}C(O)X, and by sequentially derivatizing the raw material.

### <Route A: process for producing compound wherein Q¹ or Q² is group (1)>

The compound (A) wherein Q¹ or Q² is a group (1) is represented by the following compound (1 a) or the following compound (1 b). The compound (1 a) or the compound (1 b) may be obtained by reacting a compound (13) represented by the formula R^{f}COX with a carbanion represented by the following formula (17) to obtain a compound represented by the following formula (14), and reacting the compound (14) with e.g. a compound represented by R¹NHNH₂ (hydrazine or N-substituted hydrazine).

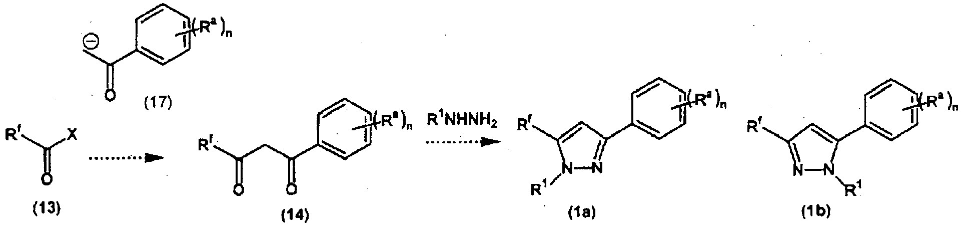

The symbols in the above formulae are as follows.

R^{f} and R¹ are synonymous with R^{f} and R¹ in the aforementioned formula (A).

X represents a halogen atom, a group obtained by removing a hydrogen atom from N-hydroxysuccinimide, a C₁₋₆ alkoxy group, a perfluorophenoxy group or a C₁₋₆ fluoroalkoxy group. As the halogen atom, a fluorine atom, a chlorine atom, a bromine atom or an iodine atom is preferred. As the C₁₋₆ alkoxy group, a methoxy group or an ethoxy group is preferred. As the C₁₋₆ fluoroalkoxy group, a 2-trifluoroethoxy group is preferred.

The R^{a} group is a hydroxy group, an amino group, a carboxy group, a halogen atom or a trifluoromethyl group.

n is an integer of from 0 to 5.

Examples of the compound (13) represented by the formula R^{f}COX include perfluoro(1-(1-propoxy)propionic acid) fluoride, perfluoro(1-(1-propoxy) propionate) ethyl, etc.

The carbanion (17) is a compound having a phenyl group substituted by n substituents R^{a} (where n is an integer of from 0 to 5, provided that n being 0 means "not substituted by R^{a"}), wherein substitution positions of R^{a} are not limited. When there are two or more substituents R^{a}, they may be the same groups or different groups. The carbanion (17) may be prepared by reacting an organometallic compound, an alkali metal alkoxide, an alkali metal hydride, etc. in a reaction solvent.

The organometallic compound may, for example, be an organic alkali metal compound such as lithium diisopropylamide, lithium hexamethyldisilazide, sodium hexamethyldisilazide or potassium hexamethyldisilazide. The alkali metal alkoxide may, for example, be sodium ethoxide or potassium t-butoxide. The alkali metal hydride may, for example, be sodium hydride.

The reaction solvent to be used in the preparation of the carbanion represented by the formula (17) may, for example, be an ether solvent such as tetrahydrofuran, diethyl ether, cyclopentyl methyl ether or 1,4-dioxane; or a hydrocarbon solvent such as hexane, pentane, benzene or toluene. Such reaction solvents may be used alone or in combination.

For the reaction to obtain the compound (14) by reacting the compound (13) and the compound (17), it is possible to employ techniques and reaction conditions which are commonly known in similar reactions.

Also for the reaction of the compound (14) with the compound represented by R¹NHNH₂, it is possible to employ conditions commonly known in similar reactions, as they are. For example, it is possible to refer to the method disclosed in WO2005/042468. Here, the compound (1a) and the compound (1 b) are in the relationship of isomers, and at least one of them can be obtained.

The compound (1 a) or the compound (1 b) wherein n is from 1 to 5, has a phenyl group substituted by R^{a} group(s). When R^{a} is a halogen atom, a fluorine atom is preferred. The phenyl group substituted by such R^{a} may, for example, be an o-fluorophenyl group, a m-fluorophenyl group, a p-fluorophenyl group, an o-trifluoromethylphenyl group, a m-trifluoromethylphenyl group, a p-trifluoromethylphenyl group, an o-fluoro-p-trifluoromethylphenyl group, an o,o-difluoro-p-trifluoromethylphenyl group, an o-hydroxyphenyl group, a m-hydroxyphenyl group, a p-hydroxyphenyl group, an o-aminophenyl group, a m-aminophenyl group, a p-aminophenyl group, an o-carboxyphenyl group, a m-carboxyphenyl group, or a p-carboxyphenyl group.

By changing the R^{a} group(s) in the carbanion (17), it is possible to obtain the compound (1 a) or the compound (1 b) with the desired R^{a} group(s).

As the compound represented by the formula (A) which has a group (1), a compound (1 b) wherein Q¹ is a hydrogen atom and Q² is a group (1), is preferred.

### <Route A: process for preparing compound wherein Q¹ or Q² is group (2)>

The compound (A) wherein Q¹ or Q² is a group (2) is the following compound (2a) or the following compound (2b). The compound (2a) or the compound (2b) may be obtained by reacting a compound (13) represented by the formula R^{f}COX, and a carbanion represented by the following formula (17') to obtain a compound (14'), then, reacting the compound (14') and a compound selected from HC(OR')₃, (CH₃O)₂CHNR"₂ and (CH₃CH₂O)₂CHNR"₂ to obtain a compound (16), and then, reacting the compound (16) and a compound represented by R¹NHNH₂ (hydrazine or N-substituted hydrazine).

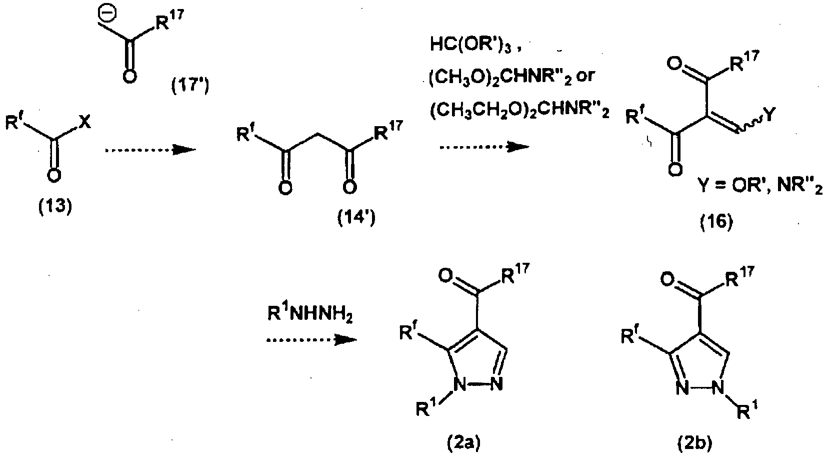

The symbols in the above formulae are as follows.

R^{f}, R¹ and X are as defined above.

R¹⁷ is a C₁₋₆ alkyl group, a phenyl group, or a C₁₋₆ alkyl group or a phenyl group in which at least one hydrogen atom bonded to a carbon atom of the group is each independently substituted by a group selected from an amino group, a hydroxy group, a halogen atom, a C₁₋₆ alkoxy group and a trifluoromethyl group. The C₁₋₆ alkyl group and the C₁₋₆ alkoxy group in R¹⁷ are, respectively, the same as these groups in R¹.

Y is a group represented by -OR' or NR", and such R' and R" each independently represent a C₁₋₃ alkyl group.

R¹⁷ is preferably a C₁₋₃ alkyl group such as a methyl group, an ethyl group or a propyl group; a C₁₋₃ alkyl group in which a hydrogen atom of the group is substituted by an alkyl group, a C₁₋₆ alkoxy group or an amino group, such as a 2-methoxyethyl group, a 2-methylaminoethyl group or a 2-dimethylaminoethyl group; a phenyl group; a phenyl group substituted by a fluorine atom such as an o-fluorophenyl group, a m-fluorophenyl group or a p-fluorophenyl group; a phenyl group substituted by a trifluoromethyl group such as an o-trifluoromethylphenyl group, a m-trifluoromethylphenyl group or a p-trifluoromethylphenyl group; a phenyl group substituted by a hydroxy group such as an o-hydroxyphenyl group, a m-hydroxyphenyl group or a p-hydroxyphenyl group; a phenyl group substituted by an amino group, such as an o-aminophenyl group, a m-aminophenyl group or a p-aminophenyl group; a phenyl group substituted by a carboxy group, such as an o-carboxyphenyl group, a m-carboxyphenyl group or a p-carboxyphenyl group; etc.

The method to obtain the compound (14') by reacting the compound (13) and the carbanion (17') can be carried out in the same manner as the method to obtain the compound (14) by reacting the above compound (13) and the carbanion (17).

Then, the compound (14') is reacted with HC(OR')₃, (CH₃O)₂CHNR"₂ or (CH₃CH₂O)₂CHNR"₂ to obtain a compound (16).

The reaction to obtain the compound (16) by reacting the compound (14 ') and HC(OR')₃ can be carried out without a solvent or in an organic solvent. As the organic solvent, acetic acid or acetic anhydride may, for example, be used, and the reaction may be carried out by adding a Lewis acid such as zinc chloride or tin chloride. The reaction temperature is preferably from room temperature (25°C) to about 200°C, particularly preferably from 100°C to 160°C.

The reaction to obtain the compound (16) by reacting the compound (14') and (CH₃O)₂CHNR"₂ or (CH₃CH₂O)₂CHNR"₂, can be carried out without a solvent or in an organic solvent. As the organic solvent, an inert solvent such as benzene, toluene, tetrahydrofuran or dioxolane may be mentioned. The reaction temperature is preferably about from 0 to 200°C, particularly preferably from 0°C to 100°C.

Then, the compound (16) and a compound represented by R¹NHNH₂ are reacted to obtain at least one compound selected from the compound (2a) and the compound (2b). The reaction can be carried out in an organic solvent. The organic solvent may, for example, be an inert solvent such as benzene, toluene, methylene chloride, acetonitrile, tetrahydrofuran, dioxolane, N, N-dimethylformamide or N,N-dimethylacetamide.

It is preferred to use R¹NHNH₂ in an amount of from 0.5 to 10 times by mole to compound (16). The reaction temperature is preferably from -10°C to 100°C, particularly preferably from 0°C to 40°C. The reaction is preferably carried out in an atmosphere of an inert gas such as nitrogen or argon. The pressure is usually preferably under the atmospheric pressure or under a slightly pressurized condition at a level of 0.11 MPa (gauge pressure).

The compound (2a) and the compound (2b) are preferably the following compound (2a-1) and the following compound (2b-1), wherein R¹⁷ is a phenyl group, or a phenyl group wherein at least one hydrogen atom bonded to a carbon atom of the phenyl group is each independently substituted by a group selected from a hydroxy group, an amino group, a halogen atom, a C₁₋₆ alkoxy group and a trifluoromethyl group. The production route for the compound (2a-1) and the compound (2b-1) may be represented by the following formulae.

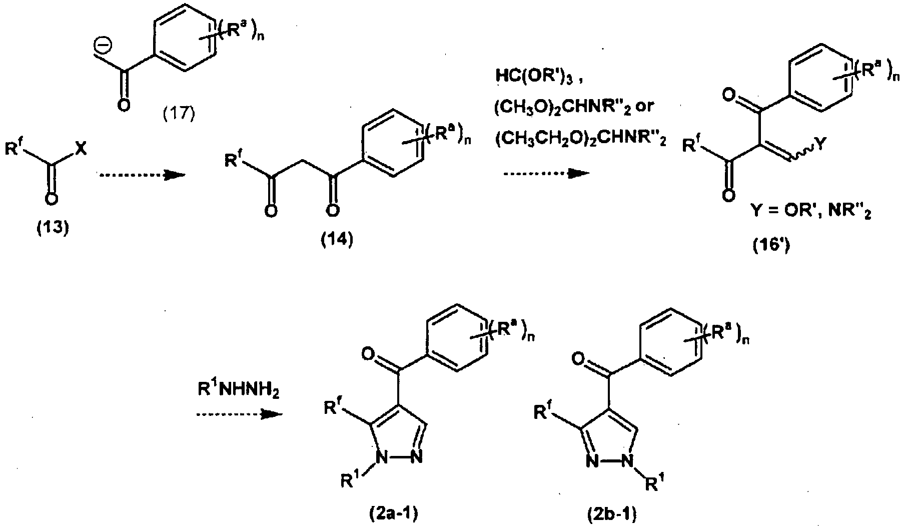

The symbols in the formulae are as follows.

R^{f}, R¹, X, R^{a}, n, Y, R' and R" are as defined above.

As the reaction conditions for the compound (2a-1) and the compound (2b-1), the conditions for the compound (2a) and the compound (2b) may be employed as they are. As the compound represented by the formula (A), which has a group (2), the compound (2b-1) wherein Q¹ is a group (2) and Q² is a hydrogen atom, is preferred.

### <Route B: method for producing compound wherein Q¹ or Q² is any one of groups (3) to (7)>

Compounds wherein Q² is a group (3), are the following compound (3a) and the following compound (3b), and may be obtained by reacting the compound (13) represented by R^{f}COX and a compound represented by the formula (18) to obtain a compound represented by the formula (19), and then, reacting the compound (19) and a compound represented by R¹NHNH₂.

Compounds wherein Q² is a group (4), are the following compound (4a) and the following compound (4b), and may be obtained by hydrolyzing the compound (3a) and the compound (3b).

Compounds wherein Q² is a group (5), are the following compound (5a) and the following compound (5b), and may be obtained by forming the compound (4a) and the compound (4b) by a mixed acid anhydride agent into a mixed acid anhydride, which is then converted by a metal azide compound to a keto azide, which is further reacted with an alcohol represented by R¹⁶OH while being subjected to a rearrangement reaction.

Compounds wherein Q² is a group (6), are the following compound (6a) and the following compound (6b), and may be obtained by reacting the compound (5a) and the compound (5b) with an acid.

Compounds wherein Q² is a group (7), are the following compound (7a) and the following compound (7b), and may be obtained by reacting the compound (6a) and the compound (6b) with R²²C(O)Cl.

Such a production route may be shown by the following formulae.

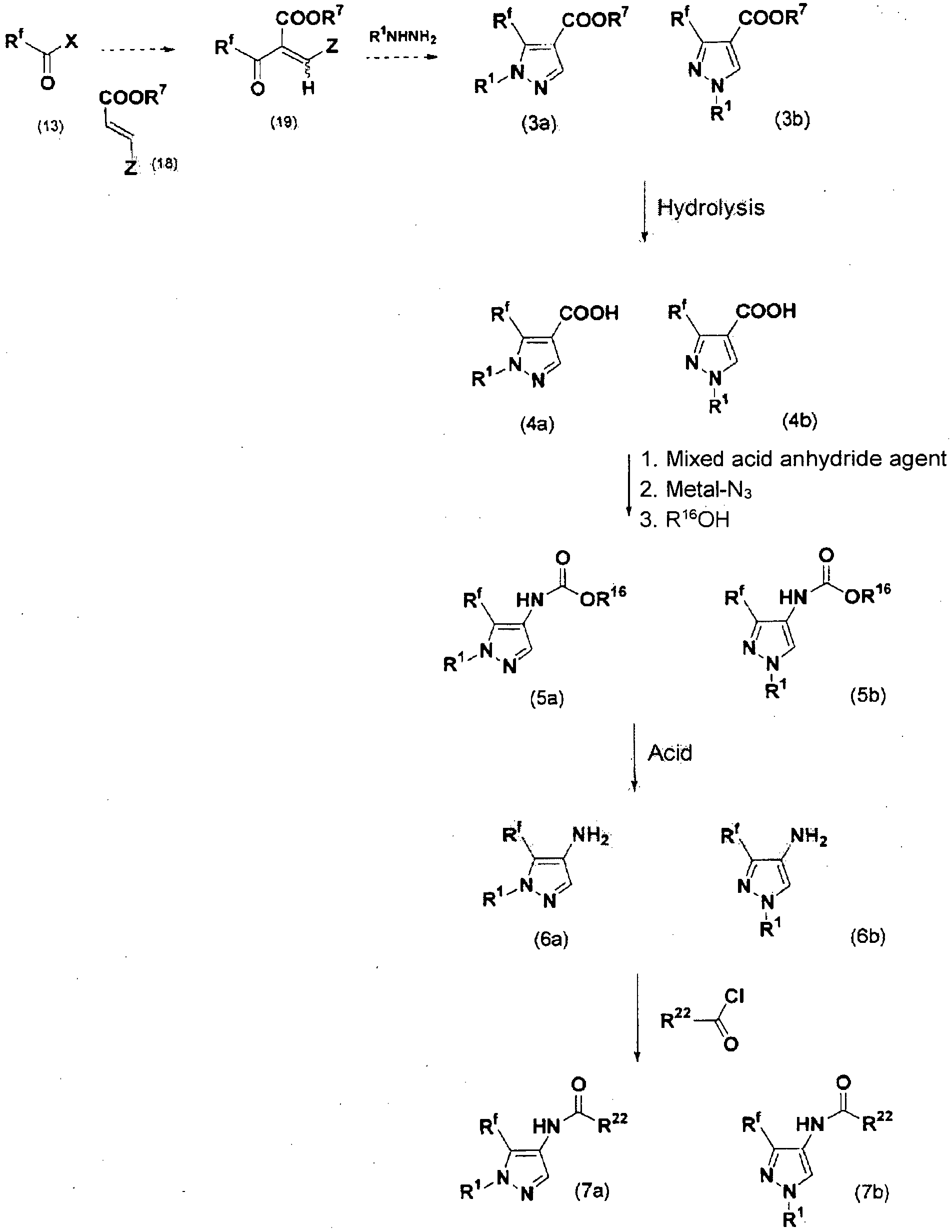

In the formulae of the above production route, R^{f}, R¹ and X are as defined above.

R⁷ is a C₁₋₆ alkyl group, an aralkyl group, a phenyl group, or a phenyl group in which at least one hydrogen atom of the phenyl group is each independently substituted by a group selected from an amino group, a substituted amino group, a C₁₋₆ alkoxy group and a halogen atom.

Z is a group represented by -OR' or NR"₂, and such R' and R" are each independently a C₁₋₃ alkyl group.

R¹⁶ is a C₁₋₆ alkyl group, or a C₁₋₆ alkyl group in which at least one hydrogen atom bonded to a carbon atom of the alkyl group, is substituted by a halogen atom.

R²² is a C₁₋₆ alkyl group, a phenyl group, an aralkyl group, or a C₁₋₆ alkyl group or a phenyl group in which at least one hydrogen atom bonded to a carbon atom of the group, is each independently substituted by a group selected from an amino group, a hydroxy group, a halogen atom and a trifluoromethyl group.

The reaction to obtain the compound (19) by reacting the compound (13) represented by R^{f}COX and the compound (18) may be carried out in the presence of a base in an organic solvent.

The organic solvent may, for example, be an inert solvent such as benzene, toluene, methylene chloride, acetonitrile, tetrahydrofuran, dioxolane, N, N-dimethylformamide or N,N-dimethylacetamide. The amount of the compound (18) is preferably from 0.5 to 10 mol to 1 mol of the compound (13). The reaction temperature is preferably from -10°C to 100°C, particularly preferably from 0°C to 40°C.

The above reaction is conducted in an atmosphere of an inert gas such as nitrogen or argon, usually under the atmospheric pressure or under a slightly pressurized condition at a level of about 0.11 MPa (gauge pressure).

As the base, a tertiary amine such as trimethylamine or tributylamine, or a pyridine such as pyridine or 2,6-dimethyl-pyridine, is preferred. Among them, triethylamine or pyridine is particularly preferred as the base to be used for the production.

In the case where R⁷ of the compound (18) is a C₁₋₆ alkyl group, the same group as R¹ may be mentioned. As the aralkyl group, a benzyl group may be mentioned. The substituted amino group is an amino group in which at least one hydrogen atom is substituted, and the substituent may be a methyl group, an ethyl group, a propyl group, a butyl group, a phenyl group, a benzyl group, a p-methoxybenzyl group or a fluorobenzyl group, and in particular, a methyl group or a benzyl group is preferred.

As R⁷, a C₁₋₆ alkyl group, an aralkyl group or a substituted aralkyl group is preferred, and a methyl group, an ethyl group, a benzyl group or a p-methoxybenzyl group is particularly preferred.

As the compound (18), specifically methyl β-(dimethylamino) acrylate, ethyl β-(diethyl) acrylate or ethyl β-methoxy acrylate, may be mentioned.

The compound (19) obtained by the reaction of the compound (13) and the compound (18), is then reacted with the compound represented by the formula R¹NHNH₂, to obtain the compound (3a) and the compound (3b) wherein Q¹ or Q² is a group (3).

Then, the compound (4a) and the compound (4b) are obtained by hydrolyzing the compound (3a) and the compound (3b). For the hydrolysis, e.g. an alcohol for dissolving the compound (3a) and the compound (3b) is used as a solvent, and sodium hydroxide is added thereto, followed by heating and refluxing, evaporation of the solvent, and the extraction, to hydrolyze the -COOR⁷ structure, to obtain the compound (4a) and the compound (4b) wherein Q¹ or Q² is a group (4).

Then, the compound (4a) and the compound (4b) are formed by a mixed acid anhydride agent into a mixed acid anhydride, then reacted with a metal azide compound (Metal-N₃) to obtain a keto azide, which is further reacted with an alcohol represented by R¹⁶OH while being subjected to a rearrangement reaction, to obtain the compound (5a) and the compound (5b) wherein Q¹ or Q² is a group (5). As R¹⁶, a C₁₋₄ alkyl group, or a C₁₋₄ alkyl group substituted by a chlorine atom, is preferred, and a t-butyl group or a 2,2,2-trichloroethyl group, is more preferred.

As the mixed acid anhydride agent, it is possible to use, for example, ethyl chloroformate or methyl chloroformate, and ethyl chloroformate is preferred. As the metal azide compound, it is possible to use sodium azide.

Then, by reacting the compound (5a) and the compound (5b) with an acid such as hydrochloric acid or sulfuric acid, it is possible to obtain the compound (6a) and the compound (6b) wherein Q¹ or Q² is a group (6), i.e. an amino group. It is preferred to carry out such a reaction in a reaction solvent, and as the reaction solvent, dioxane, diethyl ether, t-butyl methyl ether, cyclopentyl methyl ether, or the like, is preferred, and dioxane or cyclopentyl methyl ether is particularly preferred.

Then, by reacting the compound (6a) and the compound (6b) with the compound represented by the formula R²²C(O)Cl, it is possible to synthesize the compound (7a) and the compound (7b) wherein Q¹ or Q² is a group (6).

R²² is preferably a phenyl group, a phenyl group substituted by a fluorine atom, a phenyl group substituted by a trifluoromethyl group, or a phenyl group substituted by a fluorine atom and a trifluoromethyl group, particularly preferably a phenyl group, or a 2-fluoro-4-trifluoromethylphenyl group.

As the compound represented by the formula R²²C(O)Cl, benzoyl chloride, 2-fluorobenzoyl chloride, 2-fluoro-4-trifluoromethylbenzoyl chloride or the like, is preferred, and benzoyl chloride or 2-fluoro-4-trifluoromethylbenzoyl chloride is particularly preferred.

As the compound wherein Q¹ or Q² is any of groups (3) to (7), preferred is a compound wherein Q¹ is any of groups (3) to (7), and Q² is a hydrogen atom.

In the method for producing a compound in the present invention, of which a group is substituted by a functional group such as a hydroxyl group, a carboxyl group or an amino group, if the protection or deprotection of such a group is required, a known method (see e.g. Protective Groups in Organic Synthesis, P.G.M. Wuts & T.W. Greene, Jhon wiley & Sons, Inc.) may be used.

Further, it is possible to isolate the desired compound as post-treatment after the reaction, by carrying out a common operation in organic synthesis. As such an isolation procedure, activated carbon treatment, distillation, recrystallization, crystallization, column chromatography or the like may be carried out as the case requires.

Among the compounds of the present invention (A), particularly preferred compounds will be shown below.

In the following formulae, C₃F₇O- is CF₃CF₂CF₂O-, and tBu means a t-butyl group.

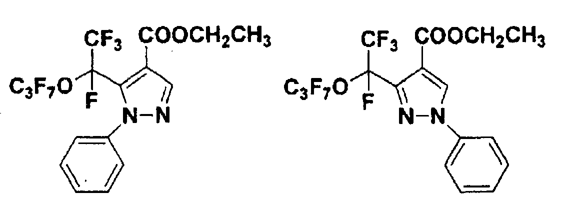

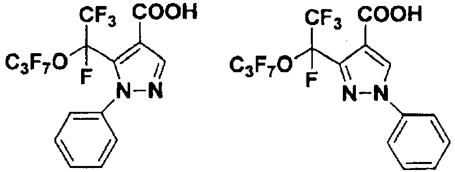

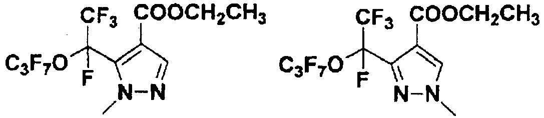

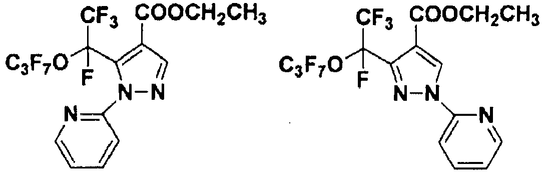

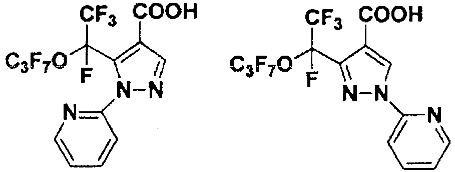

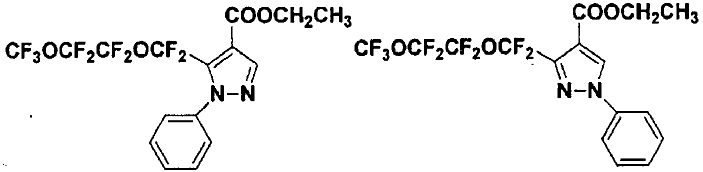

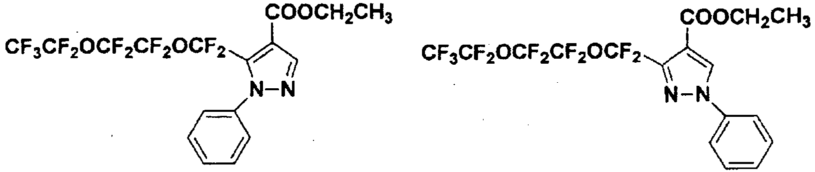

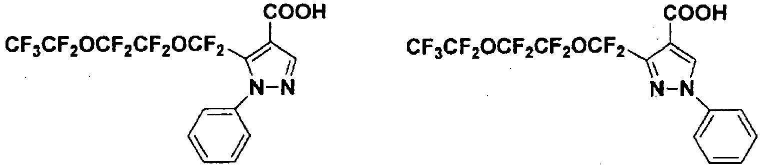

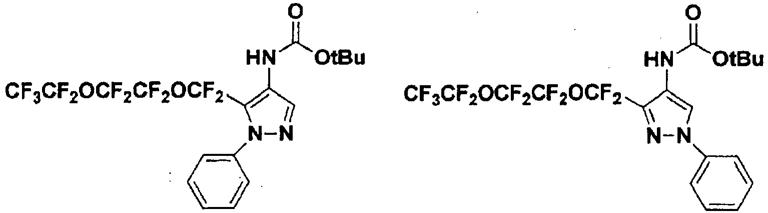

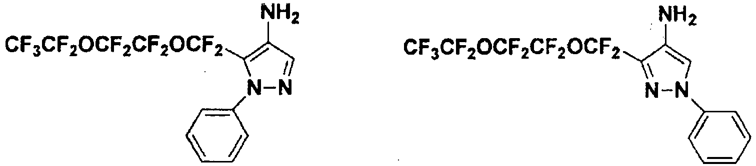

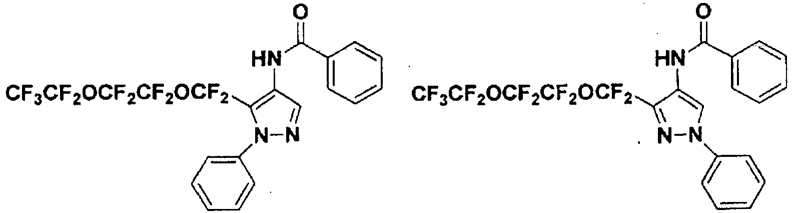

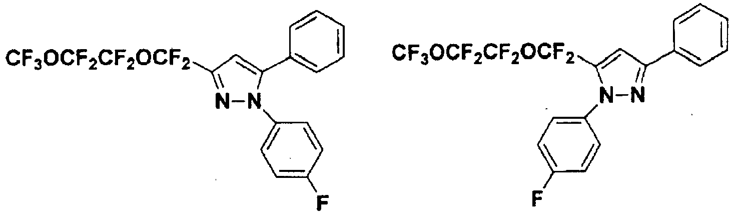

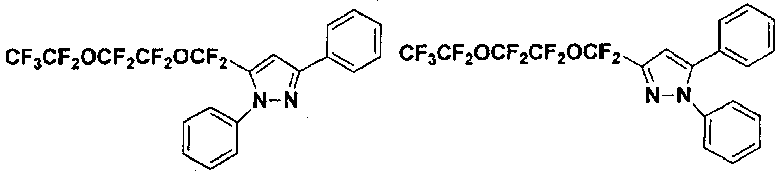

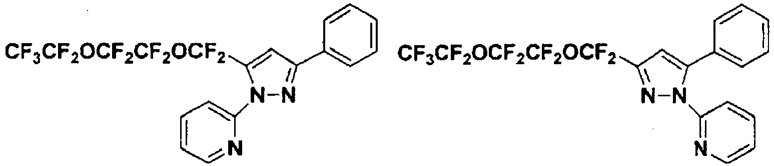

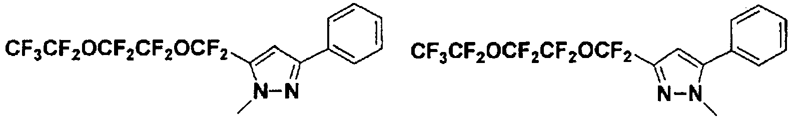

According to the compound of the present invention, a novel pyrazole derivative is provided wherein to two carbon atoms among the three carbon atoms of a pyrazole ring, a fluorinated alkyl group having an ethereal oxygen atom and a specific substituent are bonded, and to the remaining one carbon atom, a hydrogen is bonded. A perfluoroalkyl group having an ethereal oxygen atom is a group capable of being bended and having a hydrophobic property. Thus, when used as a pharmaceutical or an agrochemical, the compound of the present invention becomes capable of bonding to an active site and can exhibit a physiological activity. Further, by converting each substituent represented by Q¹ or Q², i.e. an ester bond, an amide bond or an amino group formed by the conversion reaction of a carboxy group, to an optional functional group, derivatization to various pyrazole compounds becomes possible, which are expected to have high pharmacological activities as new pharmaceuticals or agricultural chemicals.

### EXAMPLES

Now, the present invention will be described with reference to Examples. However, the invention is not to be construed as being limited to these Examples. In the formula, C₃F₇O- is CF₃CF₂CF₂O-, and tBu means a t-butyl group. Nuclear magnetic resonance spectrum (NMR) was measured by using JNM-AL300 manufactured by JEOL Ltd.

### <Synthesis Example 1>

Under a nitrogen atmosphere, ethyl β-dimethylaminoethyl acrylate (1.43 g) was dissolved in toluene (10 ml), and pyridine (0.8 g) was added at room temperature. Perfluoro(1-(1-propoxy) propionic acid) fluoride (perfluoro(2-methyl-3-oxa-hexanoyl) fluoride) (3.2 g) was dropwise added thereto, followed by stirring for 10 hours at the same temperature. Water (20 ml) was added to the reaction mixture, and the organic phase was separated. Further, the aqueous phase was extracted with toluene (10 ml), and the extract was combined with the previous organic phase. This organic phase was dried over anhydrous magnesium sulfate, and the solvent was distilled off to obtain 4.5 g of a compound represented by the following formula.

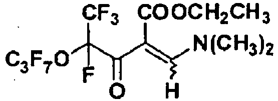

¹H-NMR (300 MHz, CDCl₃) δ=1.31 (t, J=7.2 Hz, 3 H), 3.07 (s, 3 H), 3.31 (s, 3 H), 4.17 (q, J=7.2 Hz, 2 H), 7.67 (s, 1 H).

### <Example 1: Preparation Example of compound (3a-1) and compound (3b-1)>

The compound (1.13 g) obtained in Synthesis Example 1 was dissolved in acetonitrile (4 ml), and under a nitrogen atmosphere, phenylhydrazine (0.27 g) was added at room temperature, followed by stirring for 4 hours at the same temperature. The solvent was distilled off under reduced pressure, followed by purification by silica gel column chromatography (hexane/ethyl acetate=10/1 (volume ratio, hereinafter the same)) to obtain 0.73 g of a mixture of the following compounds.

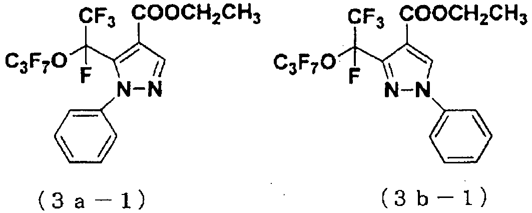

¹H-NMR (300 MHz, CDCl₃) δ=1.33 (t, J=7.2 Hz, 3 H), 4.29 (q, J=7.2 Hz, 1 H, regioisomer), 4.30 (q, J=7.2 Hz, 1 H, regioisomer), 7.3-7.4 (m, 2 H), 7.4-7.6 (m, 3 H), 8.20 (s, 0.5 H, positional isomers), 8.21 (s, 0.5 H, positional isomers).

¹⁹F-NMR (300 MHz, CDCl₃, CCl₃F standard) δ=-81.6 (3F), -82.1 (1 F), -82.3 (3F), -84.4 (1F), -120.1 (1F), -129.9 (2F).

### <Example 2: Preparation Example of compound (4a-1) and compound (4b-1)>

The mixture (0.43 g) of the compounds obtained in Example 1 was dissolved in ethanol (3 ml), and an aqueous solution (3 ml) of sodium hydroxide (60 mg) was added at room temperature, followed by heating and refluxing at 105°C. After 1.5 hours, the solvent was distilled off under reduced pressure, water (10 ml) was added, the pH was adjusted to about 2 with 10% sulfuric acid, followed by extraction with methylene chloride (10 ml). The solvent was distilled off under reduced pressure, to obtain 0.40 g of a mixture of the following compounds.

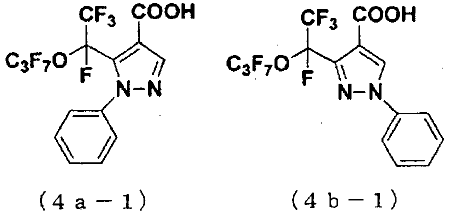

¹H-NMR (300 MHz, CDCl₃) δ=7.3-7.4 (m, 2 H), 7.4-7.5 (m, 3 H), 8.30 (s, 1 H, regioisomer).

¹⁹F-NMR (300 MHz, CDCl₃, CCl₃F standard) δ=-80.4 (1 F), -81.9 (3F), -82.9 (3F), -85.6 (1F), -121.1 (1F), -130.0 (2F).

### <Example 3: Preparation Example of compound (3a-2) and compound (3b-2)>

The compound (0.91 g) obtained in Synthesis Example 1 was dissolved in acetonitrile (4 ml), and under a nitrogen atmosphere, methylhydrazine (0.22 g) was added at room temperature, followed by stirring for 24 hours at the same temperature. The solvent was distilled off under reduced pressure, followed by purification by silica gel column chromatography (hexane/ethyl acetate=10/1) to obtain 0.12 g of a mixture of the following compounds.

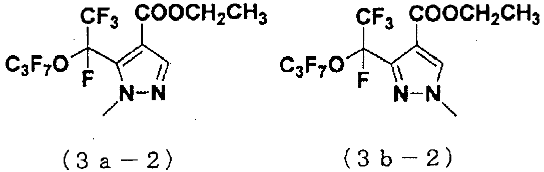

¹H-NMR (300 MHz, CDCl₃) δ=1.33 (t, J=7.2 Hz, 3 H), 4.08 (s, 1.5 H, regioisomer), 4.10 (S, 1. 5 H), 4.26 (q, J=7.2 Hz, 1 H, regioisomer), 4.31 (q, J=7.2Hz, 1 H, regioisomer), 7.99 (s, 1 H).

¹⁹F-NMR (300 MHz, CDCl₃, CCl₃F standard) δ=-81.6 (3F), -81.7 (1 F), -83.3 (3F), -84.3 (1F), -122.6 (1F), -129.8 (2F).

### <Example 4: Preparation Example of compound (3a-3) and compound (3b-3)>

The compound (0.91 g) obtained in Synthesis Example 1 was dissolved in acetonitrile (6 ml), and under a nitrogen atmosphere, 2-hydrazinopyridine (0.32 g) was added at room temperature, followed by stirring for 3.5 hours at the same temperature. The solvent was distilled off under reduced pressure, followed by purification by silica gel column chromatography (hexane/ethyl acetate=10/1) to obtain 0.25 g of a mixture of the following compounds.

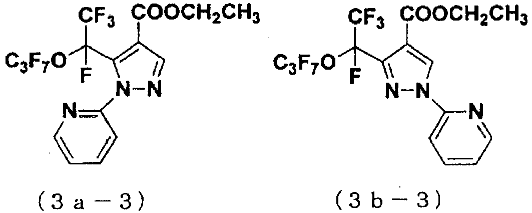

¹H-NMR (300 MHz, CDCl₃) δ=1.42 (t, J=7.2Hz, 3 H), 4.33 (q, J=7.2 Hz, 1 H, regioisomer), 4.39 (q, J=7.2 Hz, 1 H, regioisomer), 7.4-7.5 (m, 2 H), 7.48-7.9 (m, 1 H), 8.2-8.30 (m, 1 H), 8.5-8.6 (m, 1 H).

¹⁹F-NMR (300 MHz, CDCl₃, CCl₃F standard) δ=-80.6 (3F), -81.7 (3F), -81.8 (1 F), -84.2 (1F), 117.7 (1F), -130.0 (2F).

### <Example 5: Preparation Example of compound (4a-2) and compound (4b-2)>

The mixture (0.25 g) of the compounds obtained in Example 4 was dissolved in ethanol (2 ml), and an aqueous solution (2 ml) of sodium hydroxide (60 mg) was added at room temperature, followed by heating and refluxing at 105°C. After 1 hour, the solvent was distilled off under reduced pressure, water (5 ml) was added, and the pH was adjusted to about 2 with 10% hydrochloric acid, followed by extraction with methylene chloride (10 ml). The solvent was distilled off under reduced pressure, to obtain 0.22 g of a mixture of the following compounds.

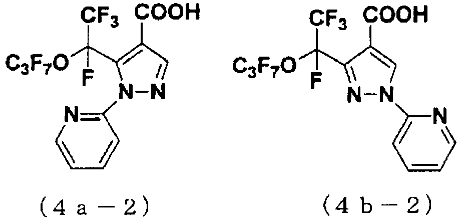

¹H-NMR (300 MHz, CDCl₃) δ=7.3-7.5 (m, 2 H), 7.8-7.9 (m, 3 H), 8.3-8.4 (m, 1 H), 8.5-8.6 (m, 1 H), 10.8-11.3 (m, 1 H).

¹⁹F-NMR (300 MHz, CDCl₃, CCl₃F standard) δ=-80.8 (1 F), -81.2 (3F), -81.4 (3F), -85.1 (1 F), -119.1 (1F), -130.0 (2F).

### <Synthesis Example 2>

In Synthesis Example 1, except that instead of perfluoro(1-(1-propoxy) propionic acid) fluoride (perfluoro(2-methyl-3-oxa-hexanoyl) fluoride), perfluoro(3,6-dioxa heptanoyl) chloride (1.4 g) was used, the reaction was carried out for 0.5 hour under the same conditions, to obtain 0.6 g of the following compound.

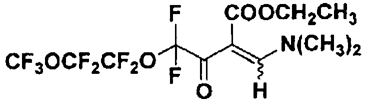

¹H-NMR (300 MHz, CDCl₃) 5=1.37 (t, J=7.2 Hz, 3 H), 2.87 (s, 3 H), 3.31 (s, 3 H), 4.30 (q, J=7.2 Hz, 2 H), 7.70 (s, 1 H).

### <Example 6: Preparation Example of compound (3a-4) and compound (3b-4)>

The compound (0.6 g) obtained in Synthesis Example 2 was dissolved in acetonitrile (5 ml), and under a nitrogen atmosphere, phenylhydrazine (0.42 g) was added at room temperature, followed by stirring for 1 hour at the same temperature. The solvent was distilled off under reduced pressure, followed by purification by silica gel column chromatography (hexane/ethyl acetate=10/1) to obtain 0.06 g of a mixture of the following compounds.

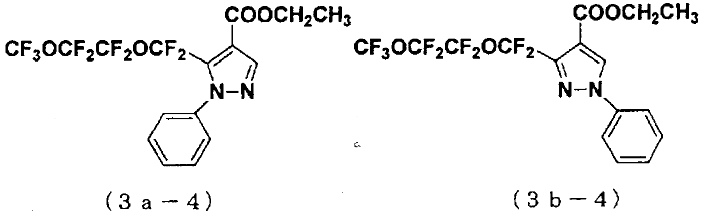

¹H-NMR (300 MHz, CDCl₃) δ=1.38 (t, J=7.2 Hz, 3 H), 4.39 (q, J=7.2 Hz), 7.2-7.6 (m, 5H), 8.07(s, 1 H).

¹⁹F-NMR (300 MHz, CDCl₃, CCl₃F standard) δ=-55.5 (3F), -60.0 (2F), -89.2 (2F), -91.2 (2F).

### <Synthesis Example 3>

In Synthesis Example 1, except that instead of perfluoro(1-(1-propoxy)propionic acid) fluoride (perfluoro(2-methyl-3-oxa-hexanoyl) fluoride), perfluoro(3,6-dioxaoctanoyl) fluoride (1.74 g) was used, the reaction was carried out for 2 hours under the same conditions, to obtain 2.58 g of the following compound.

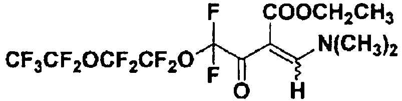

¹H-NMR (300 MHz, CDCl₃) δ=1.29 (t, J=7.2 Hz, 3 H), 2.87 (s, 3 H), 3.31 (s, 3 H), 4.15 (q, J=7.2 Hz, 2 H), 7.70 (s, 1 H).

¹⁹F-NMR (300 MHz, CDCl₃, CCl₃F standard) δ=-74.2 to -74.3 (2F), -86.2 to -86.3 (3F), -87.6 to -88.1 (6F).

### <Example 7: Preparation Example of compound (3a-5) and compound (3b-5)>

The compound (0.71 g) obtained in Synthesis Example 3 was dissolved in acetonitrile (3 ml), and under a nitrogen atmosphere, phenylhydrazine (0.32 g) was added at room temperature, followed by stirring for 3.5 hours at the same temperature. The solvent was distilled off under reduced pressure, followed by purification by silica gel column chromatography (hexane/ethyl acetate=10/1) to obtain 0.46 g of a mixture of the following compounds.

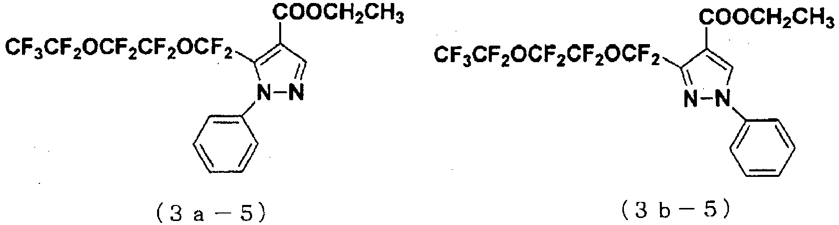

¹H-NMR (300 MHz, CDCl₃) δ=1.37 (t, J=7.2 Hz, 3 H), 4.36 (q, J=7.2 Hz, 2 H), 7.3-7.7 (m, 5 H), 8.12 (s, 1 H).

¹⁹F-NMR (300 MHz, CDCl₃, CCl₃F standard) δ=-60.1 to -60.2 (2F), -87.1 to -87.1 (3F), -88.9 to -89.0 (6F).

### <Example 8: Preparation Example of compound (4a-3) and compound (4b-3)>

The mixture (3.3 g) of the compounds obtained in Example 7 was dissolved in ethanol (10 ml), and an aqueous solution (10 ml) of sodium hydroxide (0.38 g) was added at room temperature, followed by heating and refluxing at 105°C. After 1 hour, the solvent was distilled off under reduced pressure, water (15 ml) was added, and the pH was adjusted to about 2 with 10% hydrochloric acid, followed by extraction with methylene chloride (10 ml). The solvent was distilled off under reduced pressure, to obtain 3.0 g of a mixture of the following compounds.

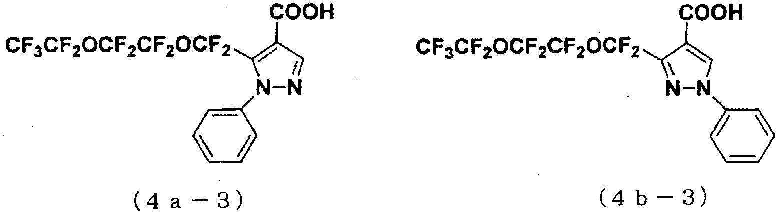

¹H-NMR (300 MHz, CDCl₃) δ=7.3-7.5 (m, 2 H), 7.5-7.6 (m, 2.97 H), 7.4-7.5 (m, 0.03 H), 7.3-7.8 (brs, 1 H), 8.20 (s, 0.99 H), 8.51 (s, 0.01 H).

¹⁹F-NMR (300 MHz, CDCl₃, CCl₃F standard) δ=-61.2 (2F), -87.2 (3F), -89.2 (6F).

### <Example 9: Preparation Example of compound (5a-1) and compound (5b-1)>

The mixture (1.35 g) of the compounds obtained in Example 8 was dissolved in acetone (15 ml), and triethylamine (0.42 g) and ethyl chloroformate (0.35 g) were added at 0°C, followed by stirring for 20 minutes. Then, at the same temperature, an aqueous solution (1.5 ml) of sodium azide (0.38 g) was added to the above reaction mixture, followed by stirring for 1 hour. Water (100 ml) was added to this reaction mixture, and the organic phase was separated. Further, the aqueous phase was extracted with toluene (10 ml), and the extract was combined with the above organic phase, followed by drying over anhydrous magnesium sulfate, and low boiling point substances were distilled off to obtain a solution of about 3 ml.

Then, a mixture of t-butyl alcohol (5 ml) and toluene (5 ml) was heated and refluxed, and the above reaction mixture was dropwise added thereto and reacted for 1.5 hours. The low boiling point substances were distilled off from the reaction mixture under reduced pressure, and the residue was purified by silica gel column chromatography (hexane/ethyl acetate=20/1) to obtain 1.1 g of a mixture of the following compounds.

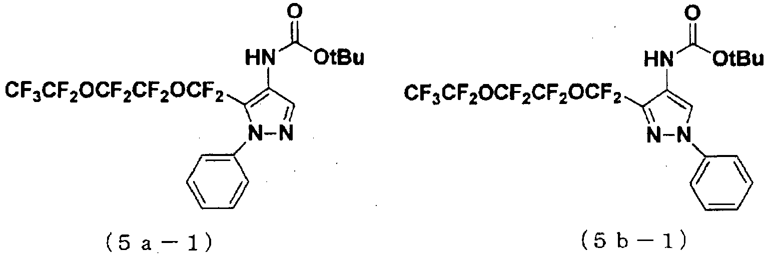

¹H-NMR (300 MHz, CDCl₃) δ=1.54 (s, 9 H), 6.56 (brs, 1 H), 7.3-7.5 (m, 5 H), 8.27 (brs, 1 H).

¹⁹F-NMR (300 MHz, CDCl₃, CCl₃F standard) δ=-59.6 (2F), -87.1 (3F), -88 to -89 (6F).

### <Example 10: Preparation Example of compound (6a-1) and compound (6b-1)>

The mixture (1.05 g) of the compounds obtained in Example 9 was dissolved in dioxane (4 ml) and 2N hydrochloric acid (4 ml), followed by heating and refluxing for 3 hours. The mixture was neutralized with a 5% sodium hydroxide solution and extracted with chloroform (5 ml), and the organic phase was concentrated to obtain 0.34 g of a mixture of the following compounds.

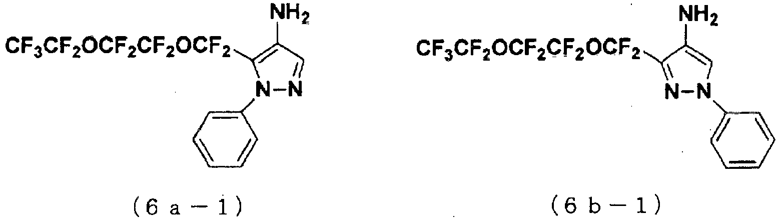

¹H-NMR (300 MHz, CDCl₃) δ=3.70 (brs, 2 H), 7.2-7.7 (m, 6 H).
¹⁹F-NMR (300 MHz, CDCl₃, CCl₃F standard) δ=-61.8 (2F), -87.0 (3F), -88.9 (6F).

### <Example 11: Preparation Example of compound (7a-1) and compound (7b-1)>

The mixture (0.06 g) of the compounds obtained in Example 10 was dissolved in pyridine (1 ml), and benzoyl chloride (0.03 g) was added at room temperature, followed by stirring for 6 hours. The reaction mixture was concentrated and purified by silica gel column chromatography (hexane/ethyl acetate=20/1) to obtain 0.02 g of a mixture of the following compounds.

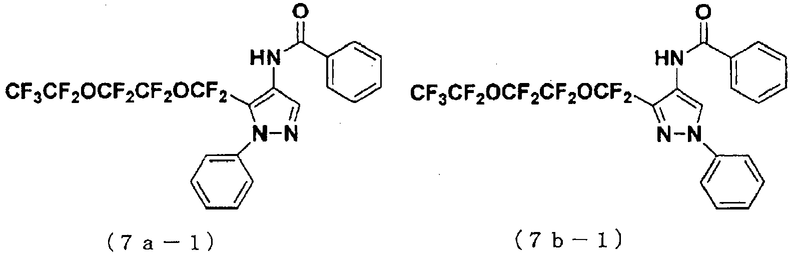

¹H-NMR (300 MHz, CDCl₃) δ=7.5-7.7 (m, 8 H), 8.2-8.3 (m, 2 H).
¹⁹F-NMR (300 MHz, CDCl₃, CCl₃F standard) δ=-60.0 (2F), -86.9 (3F), -88.6 (6F).

### <Synthesis Example 4>

Acetophenone (1.2 g) was dissolved in tetrahydrofuran (20 ml), and t-butoxy potassium (2.5 g) was added at 0°C. After stirring at room temperature for 15 minutes, perfluoro (3,6-dioxa-heptanoic acid) methyl ester (3.8 g) was added, followed by stirring for 20 hours. Water (10 ml) was added to the reaction mixture, followed by neutralization with 10% sulfuric acid. The organic phase was separated, and then, the aqueous phase was extracted with ethyl acetate (10 ml), and the extract was combined with the above organic phase, followed by drying over anhydrous sodium sulfate. The low boiling point substances were distilled off under reduced pressure, followed by purification by column chromatography (hexane/ethyl acetate=10/1), to obtain 3.4 g of the following compound.

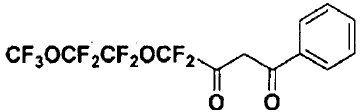

¹H-NMR (300 MHz, CDCl₃) δ=6.55 (s, 1 H), 7.5-7.7 (m, 3 H), 7.9-8.0 (m, 2 H), 14.5-15.5 (brs, 1 H).

¹⁹F-NMR (300 MHz, CDCI₃, CCl₃F standard) δ=-55.6 (3F), -79.6 (2F), -88.6 (2F), 91.1(2F).

### <Synthesis Example 5>

Acetophenone (2.4 g) was dissolved in tetrahydrofuran (40 ml), and t-butoxy potassium (5.0 g) was added at 0°C. After stirring at room temperature for 15 minutes, perfluoro(3,6-dioxaoctanoic acid) methyl ester (9.0 g) was added and stirred for 18 hours. Water (10 ml) was added to the reaction mixture, followed by neutralization with 10% sulfuric acid. The organic phase was separated, then the aqueous phase was extracted with ethyl acetate (10 ml), and the extract was combined with the above organic phase, followed by drying over anhydrous sodium sulfate. The low boiling point substances were distilled off under reduced pressure, followed by purification by column chromatography (hexane/ethyl acetate=10/1) to obtain 8.1 g of the following compound.

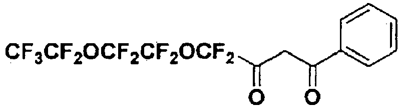

¹H-NMR (300 MHz, CDCl₃) δ=6.57 (s, 2 H), 7.4-7.5 (m, 2 H), 7.5-7.6 (m, 1 H), 7.8 -7.9 (m, 2 H).

¹⁹F-NMR (300 MHz, CDCI₃, CCl₃F standard) δ=-79.5 (2F), -87.1 (3F), -88.5 to -88.8 (2F), 89.0-89.2 (4F).

### <Example 12: Preparation Example of compound (1a-1) and compound (1b-1)>

The compound (0.40 g) obtained in Synthesis Example 4 was dissolved in ethanol (4 ml), and p-fluorophenylhydrazine (0.25 g) and concentrated sulfuric acid (0.03 ml) were added at room temperature, followed by stirring at 95°C for 2.5 hours. The low boiling point substances were distilled off under reduced pressure, and saturated sodium bicarbonate (5 ml) was added to the residue, followed by extraction with methylene chloride (10 ml). The organic phase was dried over anhydrous sodium sulfate, and the solvent was distilled off, followed by purification by silica gel column chromatography (hexane/ethyl acetate=10/1) to obtain 0.45 g of a mixture of the following compounds.

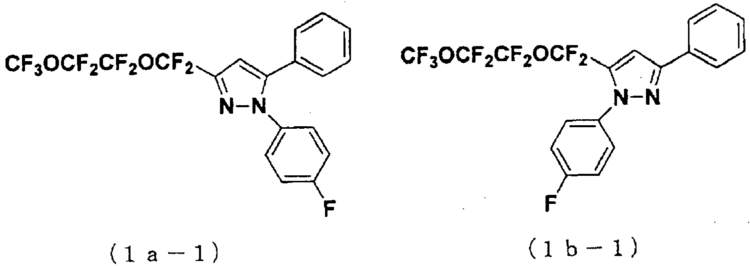

¹H-NMR (300 MHz, CDCl₃) δ=6.74 (s, 1 H), 7.1-7.6 (m, 8.55 H), 7.87 (d, J=1.8 Hz, 0. 45 H).

¹⁹F-NMR (300 MHz, CDCl₃, CCl₃F standard) δ=-55.6 (3F), -65.5 (2F), -88.7 (2F), -91.1 (2F), -112.8 (1 F).

### <Example 13: Preparation Example of compound (1 a-2) and compound (1b-2)>

The compound (0.45 g) obtained in Synthesis Example 5 was dissolved in ethanol (4 ml), and phenylhydrazine (0.16 g) and concentrated sulfuric acid (0.03 ml) were added at room temperature, followed by stirring at 100°C for 1 hour. The low boiling point substances were distilled off under reduced pressure, and saturated sodium bicarbonate (5 ml) was added to the residue, followed by extraction with chloroform (10 ml). The organic phase was dried over anhydrous sodium sulfate, and the solvent was distilled off, followed by purification by silica gel column chromatography (hexane/ethyl acetate=10/1) to obtain 0.6 g of a mixture of the following compounds.

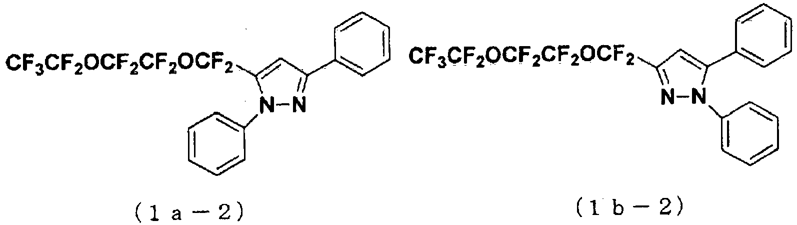

¹H-NMR (300 MHz, CDCl₃) δ=6.72 (s, 1 H), 7.3-7.5 (m, 9.5 H), 7.87 (d, J=1.8 Hz, 0. 5 H).

¹⁹F-NMR (300 MHz, CDCl₃, CCl₃F standard) δ=-65.2 (2F), -87.0 (3F), -88.6 to -88.7 (2F), -89.2 to -89.3 (4F).

### <Example 14: Preparation Example of compound (1a-3) and compound (1b-3)>

In the same manner as in Example 13 except that instead of phenylhydrazine, 2-hydrazinopyridine was used, 0.27 g of a mixture of the following compounds was obtained.

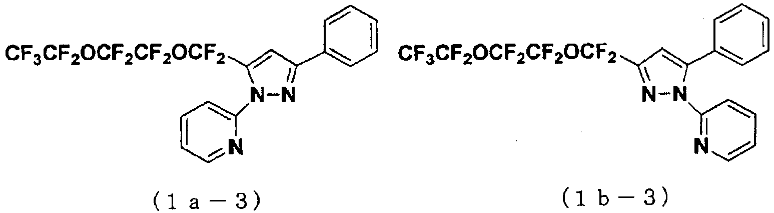

¹H-NMR (300 MHz, CDCl₃) δ=6.6-6.7 (m, 1 H), 7.2-7.4 (m, 6 H), 7.54 (d, J=7.2 Hz, 1 H), 7.81 (t, J=7.8 Hz, 1 H), 8.37 (dd, J=2.1, 4.8 Hz, 1 H).

¹⁹F-NMR (300 MHz, CDCl₃, CCl₃F standard) δ=-65.5 (2F), -87.0 (3F), -88.5 to -88.6 (2F), 89.0-89.1 (4F).

### <Example 15: Preparation Example of compound (1 a-4) and compound (1b-4)>

In the same manner as in Example 13 except that instead of phenylhydrazine, methylhydrazine was used, 0.29 g of a mixture of the following compounds was obtained.

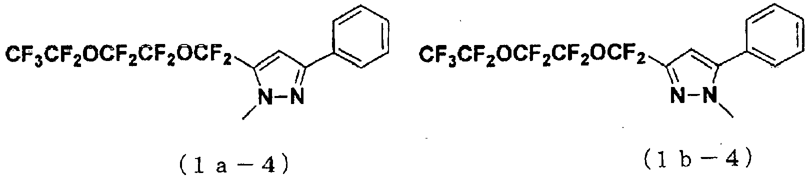

### One of the isomers:

¹H-NMR(300 MHz, CDCl₃) δ=3.95 (s, 1.71 H), 6.88 (s, 0.57 H), 7.3-7.5 (m, 2.28 H), 7.7-7.8 (m, 0.57 H).

¹⁹F-NMR (300 MHz, CDCl₃, CCl₃F standard) δ=-64.2 (2F), -87.1 (3F), -89.0 (2F), 89.1-89.3 (4F).

### One of the isomers:

¹H-NMR (300 MHz, CDCI₃) δ=4.00 (s, 1.29 H), 6.52 (s, 0.43 H), 7.3-7.6 (m, 5 H).

¹⁹F-NMR (300 MHz, CDCl₃, CCl₃F standard) δ=-65.0 (2F), -87.1 (3F), -88.9 (2F), 89.1 (4F).

### <Example 16: Preparation Example of compound (3a-6) and compound (3b-6)>

The compound (0.34 g) obtained in Synthesis Example 3 was dissolved in acetonitrile (2 ml), and under a nitrogen atmosphere, 2,6-dichloro-5-trifluoromethylphenylhydrazine (0.36 g) was added at room temperature, followed by stirring for 20 hours at the same temperature. The solvent was distilled off under reduced pressure, followed by purification by silica gel column chromatography (hexane/ethyl acetate=10/1) to obtain 0.24 g of a mixture of the following compounds.

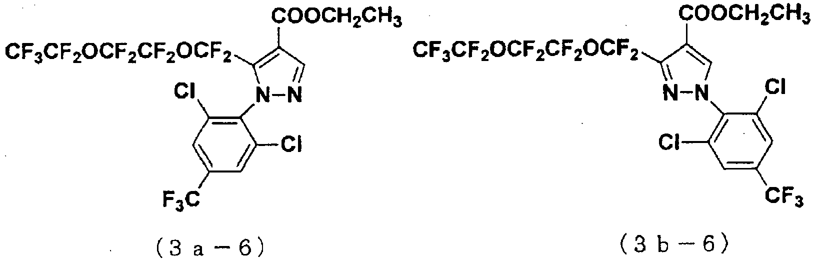

¹H-NMR (300 MHz, CDCl₃) δ=1.44 (t, J=7.2 Hz, 3 H), 4.41 (q, J=7.2 Hz), 6.84 (m, 0.5 H), 7.56 (s, 1 H), 7.77 (s, 1 H), 8.32 (s, 0.5 H).

¹⁹F-NMR (300 MHz, CDCl₃, CCl₃F standard) δ=-63.1 to -63.2 (2F), -63.9 (3F), -87.0 to -87.1 (3F), -88.7 to -89.2 (6F).

### <Example 17: Antibacterial activity test against rice Botrytis cinerea and rice blast>

Test specimens (compound (4a-2) and compound (4b-2)), (compound (4a-3) and compound (4b-3)), and reference compounds i.e. iprodione and Captan, were each independently dissolved in dimethyl sulfoxide (DMSO) and adjusted to a concentration of 10,000 ppm. The test solution was dispensed 2 µ/ml in a flat-bottomed 96 well microplate, and then, conidia of rice Botrytis cinerea (Botrytis cinerea AARF-033) cultured on glucose agar medium, suspended in glucose broth and adjusted to 1×10⁴ conidia/ml, were dispensed 198 µ/mL, followed by stirring by a micromixer. At that time, the concentrations of the test specimen and the reference compound, were 100 ppm. Thereafter, the antimicrobial activities were evaluated by stationary culturing for 4 days at 25°C.

Similarly, a suspension was prepared with respect to conidia of Pyricularia oryzae (Magnaporthe grisea) cultured in oatmeal medium, and the antimicrobial activities were evaluated by using benomyl and fluoxastrobin as reference compounds. At that time, the concentrations of the test specimen, and the reference compound were 100 ppm.

The results of the respective antibacterial activity evaluations are shown in Table 1.

**[Table 1]**

| Test specimens and reference compounds | Botrytis cinerea | Magnaporthe grisea |
|---|---|---|
| Compound (4a-2) and Compound (4b-2) | | At least 95% inhibition |
| Compound (4a-3) and Compound (4b-3) | At least 95% inhibition | At least 95% inhibition |
| Iprodione | At least 95% inhibition | |
| Captan | At least 95% inhibition | |
| Benomyl | | At least 95% inhibition |
| Fluoxastrobin | | At least 95% inhibition |

From the results in Table 1, it has been confirmed that the compound (4a-2) and the compound (4b-2), and the compound (4a-3) and the compound (4b-3), respectively have antibacterial activities equal to the reference compounds, against rice Botrytis cinerea and rice blast fungus.

### <Synthesis Example 5>

In Synthesis Example 1, except that instead of perfluoro(1-(1-propoxy)propionic acid) fluoride, perfluoro(3-oxa-pentanoyl) fluoride (2.32 g) was used, the reaction was carried out under the same conditions, to obtain the following compound.

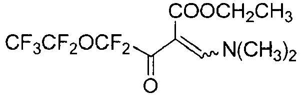

¹H-NMR (300 MHz, CDCl₃) 6=1.29 (t, J=7.2 Hz, 3 H), 2.88 (s, 3 H), 3.23 (s, 3 H), 4.21 (q, J=7.2 Hz, 2 H), 7.69 (s, 1 H).

¹⁹F-NMR (300 MHz, CDCl₃, CCl₃F standard) δ=-75.0 (2F), -87.1 (3F), -88.5 (2F).

### <Example 18: Preparation Example of compound (3a-7) and compound (3b-7)>

The compound (1.06 g) obtained in Synthesis Example 5 was dissolved in acetonitrile (5 ml), and under a nitrogen atmosphere, phenylhydrazine (0.65 g) was added at room temperature, followed by stirring for 2.5 hours at the same temperature. The solvent was distilled off under reduced pressure, followed by purification by silica gel column chromatography (hexane/ethyl acetate=10/1) to obtain 0.82 g of a mixture of the following compounds.

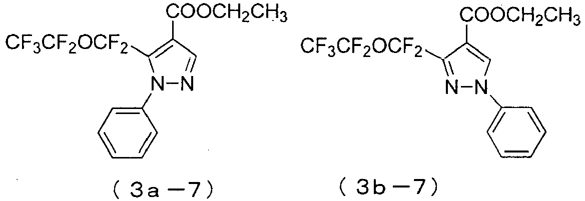

¹H-NMR (300 MHz, CDCl₃) δ=1.38 (t, J=7.2 Hz, 3 H), 4.37 (q, J=7.2 Hz, 2 H), 7.3-7.4 (m, 2 H), 7.4-7.6 (m, 3 H), 8.12 (s, 1 H).

¹⁹F-NMR (300 MHz, CDCl₃, CCl₃F standard) δ=-60.0 (2F), -87.1 (3F), -89.0 (2F).

### <Example 19: Preparation Example of compound (4a-4) and compound (4b-4)>

The mixture (0.81 g) of the compounds obtained in Example 18 was dissolved in ethanol (3 ml), and an aqueous solution (3 ml) of sodium hydroxide (0.12 g) was added at room temperature, followed by heating and refluxing at 110°C. After 1 hour, the solvent was distilled off under reduced pressure, water (5 ml) was added, and the pH was adjusted to about 2 with 10% hydrochloric acid, followed by extraction with methylene chloride (10 ml). The solvent was distilled off under reduced pressure, to obtain 0.73 g of a mixture of the following compounds.

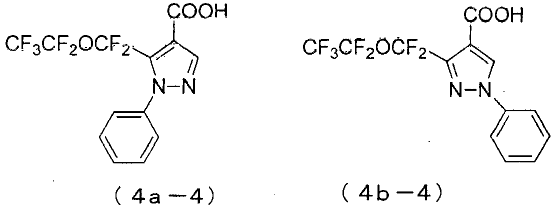

¹H-NMR (300 MHz, CDCl₃) δ=7.3-7.5 (m, 2 H), 7.5 to 7.7 (m, 3 H), 8.23 (s, 1 H), 10.58 (brs, 1 H).

¹⁹F-NMR (300 MHz, CDCl₃, CCl₃F standard) δ=-60.0 (2F), -87.1 (3F), -89.1 (2F).

### <Example 20: Preparation Example of compound (3a-8) and compound (3b-8)>

The compound (0.50 g) obtained in Synthesis Example 5 was dissolved in acetonitrile (5 ml), and under a nitrogen atmosphere, 2-hydrazinopyridine (0.31 g) was added at room temperature, followed by stirring for 2.5 hours at the same temperature. The solvent was distilled off under reduced pressure, followed by purification by silica gel column chromatography (hexane/ethyl acetate=5/1) to obtain 0.19 g of a mixture of the following compounds.

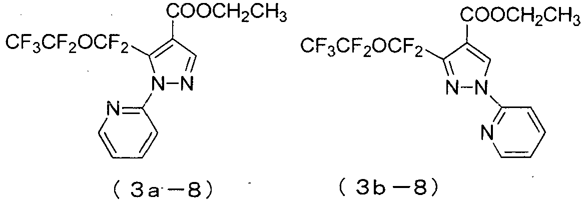

¹H-NMR (300 MHz, CDCl₃) δ=1.38 (t, J=7.2 Hz, 3 H), 4.38 (q, J=7.2 Hz, 2 H), 7.44 (dd, J=4.5, 8.1 Hz, 1 H), 7.62 (d, J=8.1 Hz, 1 H), 7.92 (t, J=8.1 Hz, 1 H), 8.13 (s, 1 H), 8.55 (d, J=4.5 Hz, 1 H).

¹⁹F-NMR (300 MHz, CDCl₃, CCl₃F standard) δ=-59.7 (2F), -87.1 (3F), -89.1 (2F).

### <Example 21: Preparation Example of compound (3a-9) and compound (3b-9)>

The compound (1.0 g) obtained in Synthesis Example 5 was dissolved in acetonitrile (5 ml), and under a nitrogen atmosphere, methylhydrazine (0.39 g) was added at room temperature, followed by stirring for 4 hours at the same temperature. The solvent was distilled off under reduced pressure, followed by purification by silica gel column chromatography (hexane/ethyl acetate=4/1) to obtain 0.12 g of a mixture of the following compounds.

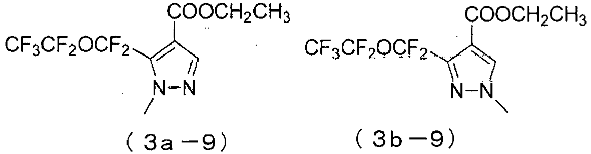

¹H-NMR (300 MHz, CDCl₃) δ=1.33 (t, J=7.2 Hz, 3 H), 4.08 (t, J=2.2 Hz, 3 H), 4.32 (J=7. 2 Hz, 2 H), 7.95 (s, 1 H).

¹⁹F-NMR (300 MHz, CDCI₃, CCl₃F standard) δ=-61.8 (2F), -87.1 (3F), -89.0 (2F).

### INDUSTRIAL APPLICABILITY

When its substituents have an ester bond, an amide bond or a carboxyl group, the novel pyrazole derivative of the present invention is useful as an intermediate for pyrazole ring compounds, since derivatization to various pyrazole compounds becomes possible by conversion reactions of such substituents. Further, it is expected to have high pharmacological activities as a drug substance for a novel medicine or agricultural chemical.

The entire disclosure of Japanese Patent Application No. 2014-003589 filed on January 10, 2014 including specification, claims, drawings and summary is incorporated herein by reference in its entirety.

## Claims

1. A compound represented by the following formula (A): 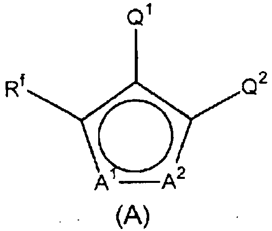 in the above formula, R^{f}, A¹, A², Q¹ and Q² have the following meanings, respectively:
R^{f}: a C₂₋₁₉ perfluoroalkyl group in which ethereal oxygen atom(s) is inserted between carbon-carbon atoms of the group, and the sum of the number of carbon atoms and the number of oxygen atom(s) in said Rf is from 3 to 20,
A¹ and A²: either one is a nitrogen atom, and the other is a nitrogen atom to which R¹ is bonded, wherein R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group is each independently substituted by a group selected from a hydroxy group, an amino group, a carboxy group and a halogen atom, (vi) a group selected from the above groups (ii) to (iv), in which at least one hydrogen atom bonded to a carbon atom of the group is each independently substituted by a C₁₋₆ halogenated alkyl group, (vii) a hydrogen atom, or (viii) a group represented by R¹²C(O)- wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group, or an aralkyl group) is inserted between carbon-carbon atoms in the alkyl group, (c) a C₁₋₆ alkoxy group, (d) a C₁₋₆ alkoxy group in which an oxygen atom is inserted between carbon-carbon atoms of the alkoxy group, (e) a phenyl, or (f) a group selected from the above groups (a) to (e), in which at least one hydrogen atom bonded to a carbon atom of the group is each independently substituted by a group selected from a hydroxy group, an amino group, a carboxy group and a halogen atom,
Q¹ and Q²: either one is a hydrogen atom, and the other is one group selected from the following (1) to (7):
(1) a phenyl group, or a phenyl group in which at least one hydrogen atom of the phenyl group is each independently substituted by a group selected from a hydroxy group, an amino group, a carboxy group, a halogen atom and a trifluoromethyl group,
(2) a group represented by R¹⁷C(O)- (wherein R¹⁷ is a C₁₋₆ alkyl group, a phenyl group, or a C₁₋₆ alkyl group or a phenyl group, in which at least one hydrogen atom bonded to a carbon atom of the group, is each independently substituted by a group selected from an amino group, a hydroxy group, a halogen atom, a C₁₋₆ alkoxy group and a trifluoromethyl group),
(3) a group represented by R⁷OC(O) (wherein R⁷ is a C₁₋₆ alkyl group, an aralkyl group, a phenyl group, or a phenyl group in which at least one hydrogen atom of the phenyl group is each independently substituted by a group selected from an amino group, a substituted amino group, a C₁₋₆ alkoxy group and a halogen atom),
(4) a carboxy group,
(5) a group represented by R¹⁶OC(O)NH- (wherein R¹⁶ is a C₁₋₆ alkyl group, or a C₁₋₆ alkyl group in which at least one hydrogen atom bonded to a carbon atom of the group is substituted by a halogen atom),
(6) an amino group,
(7) a group represented by R²²C(O)NH- (wherein R²² is a C₁₋₆ alkyl group, a phenyl group, an aralkyl group, or a C₁₋₆ alkyl group, a phenyl group or an aralkyl group, in which at least one hydrogen atom bonded to a carbon atom of the group is each independently substituted by a group selected from an amino group, a hydroxy group, a halogen atom and a trifluoromethyl group).

2. The compound according to Claim 1, wherein in the formula (A), R^{f} is a group represented by (R^{f10})(R^{f11})(R^{f12})C-O-(R^{f13})(R^{f14})C-, wherein R^{f10} to R^{f14} are each independently a C₁₋₁₂ perfluoroalkyl group, a C₁₋₁₂ perfluoroalkyl group having an ethereal oxygen atom at least either between carbon-carbon atoms or at a bond terminal, or a fluorine atom, and the sum of the number of carbon atoms and the number of oxygen atoms, is from 3 to 20.

3. The compound according to Claim 1 or 2, wherein in the formula (A), A¹ is a nitrogen atom to which said R¹ is bonded, and A² is a nitrogen atom.

4. The compound according to Claim 1 or 2, wherein the compound represented by the formula (A) is a compound represented by the following formula (1 a) or a compound represented by the following formula (1 b): 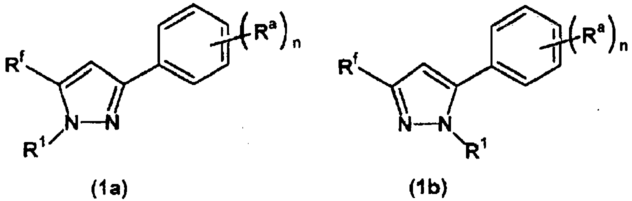 in the above formulae, the definitions of R^{f} and R¹ are the same as above, R^{a} is a hydroxy group, an amino group, a carboxy group, a halogen atom or a trifluoromethyl group, and n is an integer of from 0 to 5.

5. A method for producing at least one of a compound represented by the following formula (1 a) and a compound represented by the following formula (1 b), which is **characterized by** reacting a compound represented by the following formula (14) and a compound represented by the formula R¹NHNH₂: 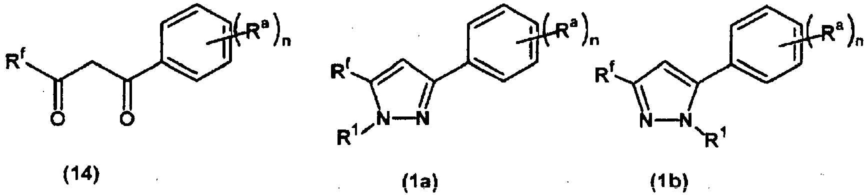 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, R^{a} is a hydroxy group, an amino group, a carboxy group, a halogen atom or a trifluoromethyl group, and n is an integer of from 0 to 5.

6. The compound according to Claim 1 or 2, wherein the compound represented by the formula (A) is a compound represented by the following formula (2a) or a compound represented by the following formula (2b): 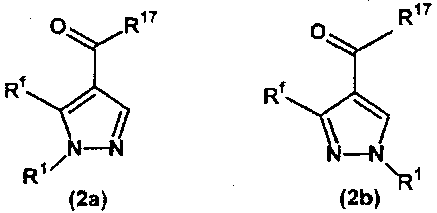 in the above formulae, the definitions of R^{f}, R¹ and R¹⁷ are the same as above.

7. A method for producing at least one of a compound represented by the following formula (2a) and a compound represented by the following formula (2b), which is **characterized by** reacting a compound represented by the following formula (16) and a compound represented by the formula R¹NHNH₂: 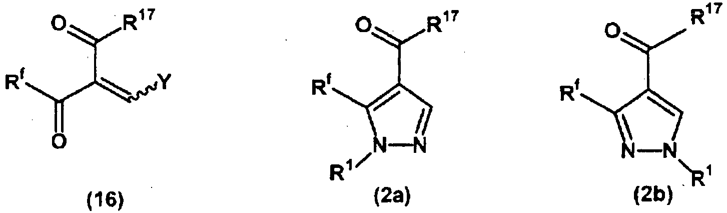 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁₋₆ halogenated alkyl group, (vii) a hydrogen atom, or (viii) a group represented by R¹²C(O)-, wherein R¹² is (a) a C₁₋₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, R¹⁷ is a C₁₋₆ alkyl group, a phenyl group, or a C₁₋₆ alkyl group or a phenyl group, in which at least one hydrogen atom bonded to a carbon atom of the group, is each independently substituted by a group selected from an amino group, a hydroxy group, a halogen atom, a C₁₋₆ alkoxy group and a trifluoromethyl group, Y is a group represented by -OR' or NR"₂, and the above R' and R" each independently represent a C₁₋₃ alkyl group.

8. The compound according to Claim 1 or 2, wherein the compound represented by the formula (A) is a compound represented by the following formula (3a) or a compound represented by the following formula (3b): 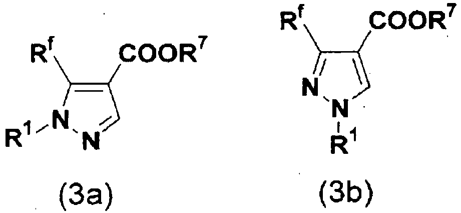 in the above formulae, the definitions of R^{f}, R¹ and R⁷ are the same as above.

9. A method for producing at least one of a compound represented by the following formula (3a) and a compound represented by the following formula (3b), which is **characterized by** reacting a compound represented by the following formula (19) and a compound represented by the formula R¹NHNH₂: 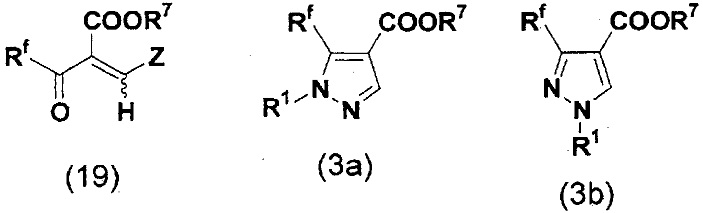 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R⁷ is a C₁-₆ alkyl group, an aralkyl group, a phenyl group, or a phenyl group in which at least one hydrogen atom of the phenyl group is each independently substituted by a group selected from an amino group, a substituted amino group, a C₁-₆ alkoxy group and a halogen atom, Z is a group represented by -OR' or NR"₂, and the above R' and R" each independently represent a C₁₋₃ alkyl group, R¹ is (i) a C₁-₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁-₆ halogenated alkyl group, (vii) a hydrogen atom, or (viii) a group represented by R¹²C(O)-, wherein R¹² is (a) a C₁-₆ alkyl group, (b) a C₁-₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁-₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom.

10. The compound according to Claim 1 or 2, wherein the compound represented by the formula (A) is a compound represented by the following formula (4a) or a compound represented by the following formula (4b): 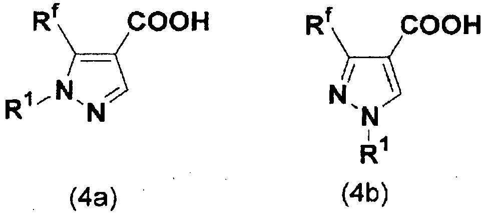 in the above formulae, the definitions of R^{f} and R¹ are the same as above.

11. A method for producing at least one of a compound represented by the following formula (4a) and a compound represented by the following formula (4b), which is **characterized by** hydrolyzing at least one of a compound represented by the following formula (3a) and a compound represented by the following formula (3b) under a basic condition: 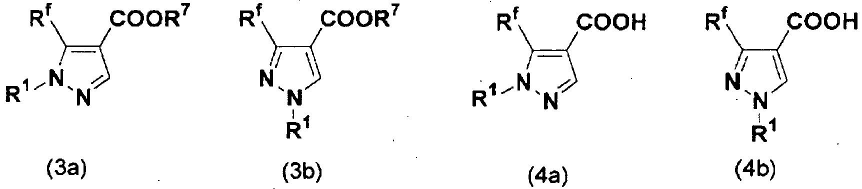 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁-₆ halogenated alkyl group, (vii) a hydrogen atom, or (viii) a group represented by R¹²C(O)-, wherein R¹² is (a) a C₁-₆ alkyl group, (b) a C₁-₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁-₆ alkoxy group, (d) a C₁-₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R⁷ is a C₁-₆ alkyl group, an aralkyl group, a phenyl group, or a phenyl group in which at least one hydrogen atom of the phenyl group is each independently substituted by a group selected from an amino group, a substituted amino group, a C₁₋₆ alkoxy group and a halogen atom.

12. The compound according to Claim 1 or 2, wherein the compound represented by the formula (A) is a compound represented by the following formula (5a) or a compound represented by the following formula (5b): 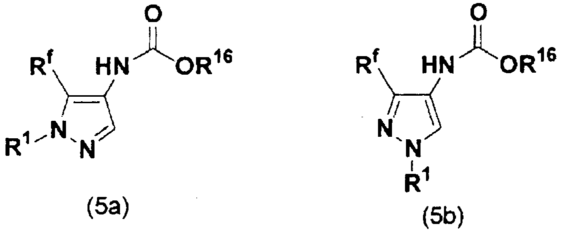 in the above formulae, the definitions of R^{f}, R¹ and R¹⁶ are the same as above.

13. A method for producing at least one of a compound represented by the following formula (5a) and a compound represented by the following formula (5b), which is **characterized in that** at least one of a compound represented by the following formula (4a) and a compound represented by the following formula (4b) is formed into a mixed acid anhydride, which is then converted by a metal azide compound to a keto azide, which is further reacted with a compound represented by R¹⁶-OH while being subjected to a rearrangement reaction: 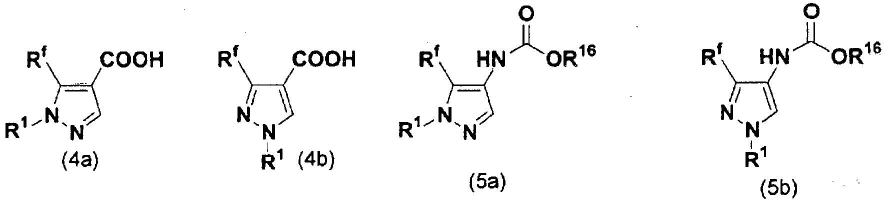 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁-₆ halogenated alkyl group, (vii) a hydrogen atom, or (viii) a group represented by R¹²C(O)-, wherein R¹² is (a) a C₁₋₆ alkyl group, (b) a C₁-₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁₋₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R¹⁶ is a C₁₋₆ alkyl group, or a C₁₋₆ alkyl group in which at least one hydrogen atom bonded to a carbon atom of the alkyl group is substituted by a halogen atom.

14. The compound according to Claim 1 or 2, wherein the compound represented by the formula (A) is a compound represented by the following formula (6a) or a compound represented by the following formula (6b): 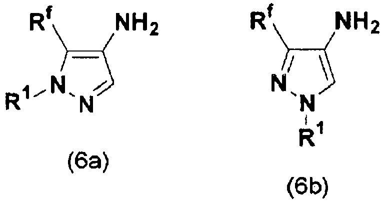 in the above formulae, the definitions of R^{f} and R¹ are the same as above.

15. A method for producing at least one of a compound represented by the following formula (6a) and a compound represented by the following formula (6b), which is **characterized by** reacting at least one of a compound represented by the following formula (5a) and a compound represented by the following formula (5b) with an acid: 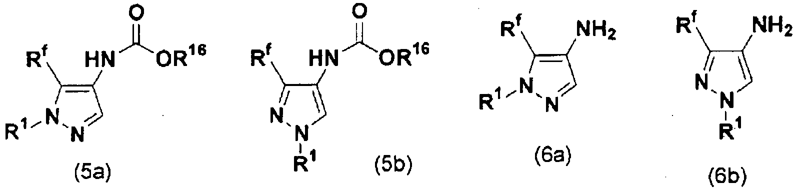 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁-₆ halogenated alkyl group, (vii) a hydrogen atom, or (viii) a group represented by R¹²C(O)-, wherein R¹² is (a) a C₁-₆ alkyl group, (b) a C₁-₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁-₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R¹⁶ is a C₁-₆ alkyl group, or a C₁-₆ alkyl group in which at least one hydrogen atom bonded to a carbon atom of the alkyl group is substituted by a halogen atom.

16. The compound according to Claim 1 or 2, wherein the compound represented by the formula (A) is a compound represented by the following formula (7a) or a compound represented by the following formula (7b): 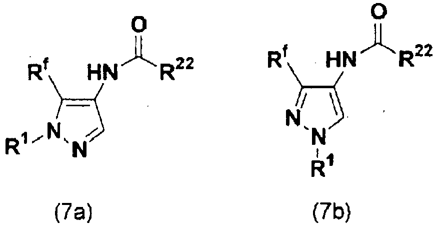 in the above formulae, the definitions of R^{f}, R¹ and R²² are the same as above.

17. A method for producing at least one of a compound represented by the following formula (7a) and a compound represented by the following formula (7b), which is **characterized by** reacting at least one of a compound represented by the following formula (6a) and a compound represented by the following formula (6b) with R²²COX (wherein X is a halogen atom, a group obtained by removing a hydrogen atom from N-hydroxysuccinimide, a C₁-₆ alkoxy group, a perfluoro phenoxy group or a C₁₋₆ fluoroalkoxy group): 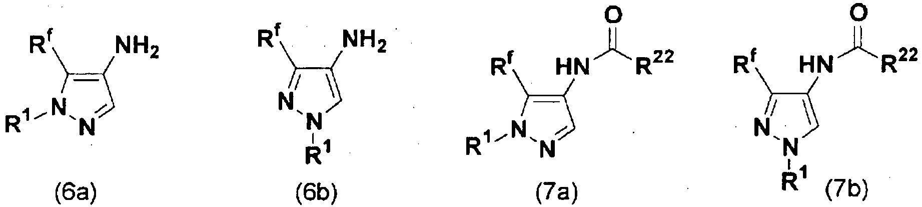 in the above formulae, R^{f} is a C₂₋₁₉ perfluoroalkyl group in which ethereal oxygen atom(s) is inserted between carbon-carbon atoms of the group, and the sum of the number of carbon atoms and the number of oxygen atom(s) in said R^{f} is from 3 to 20, R¹ is (i) a C₁₋₆ alkyl group, (ii) a phenyl group, (iii) a monovalent 5-membered ring group containing a hetero atom, (iv) a monovalent 6-membered ring group containing a hetero atom, (v) a group selected from the above groups (i) to (iv), in which at least one hydrogen atom bonded to a carbon atom of the group, is each independently substituted by a group selected from a hydroxy group, an amino group, a carboxy group and a halogen atom, (vi) a group selected from the above groups (ii) to (iv), in which at least one hydrogen atom bonded to a carbon atom of the group, is each independently substituted by a C₁-₆ halogenated alkyl group, (vii) a hydrogen atom, or (viii) a group represented by R¹²C(O)-, wherein R¹² is (a) a C₁-₆ alkyl group, (b) a C₁₋₆ alkyl group in which an oxygen atom, a sulfur atom or -NR- (wherein R is a hydrogen atom, a C₁₋₃ alkyl group, a phenyl group or an aralkyl group) is inserted between carbon-carbon atoms of the alkyl group, (c) a C₁₋₆ alkoxy group, (d) a C₁-₆ alkoxy group in which an oxygen atom is inserted between carbon-carbon atoms of the alkoxy group, (e) a phenyl group, or (f) a group selected from the above groups (a) to (e), in which at least one hydrogen atom bonded to a carbon atom of the group, is each independently substituted by a group selected from a hydroxy group, an amino group, a carboxy group and a halogen atom, and R²² is a C₁-₆ alkyl group, a phenyl group, an aralkyl group, or a C₁-₆ alkyl group, a phenyl group or an aralkyl group, in which at least one hydrogen atom bonded to a carbon atom of the group, is each independently substituted by a group selected from an amino group, a hydroxy group, a halogen atom and a trifluoromethyl group.

18. An agricultural chemical containing a compound selected from the group consisting of compounds as defined in any one of Claims 1 to 4, 6, 8, 10, 12, 14, and 16 and the pharmacologically active salts of such compounds.
